# EUROPEAN PATENT APPLICATION

(11) **EP 3 656 445 A1**
(43) Date of publication of application: **27.05.2020**
(21) Application number: 20152615.9
(22) Date of filing: 30.09.2014
(51) Int. Cl.: A61Q 19/00, A61K 8/92, A61K 8/19, A61K 8/34, A61K 8/37, A61K 8/26, A61K 8/06, A61K 8/36

(54) **COMPOSITION COMPRISING HIGH CONCENTRATIONS OF SALT**

(30) Priority: 01.10.2013 GB 201317388
(62) Divisional of application: 14781629.2
(71) Applicant: Cosmetic Warriors Limited, Dorset BH15 1AB (GB)
(72) Inventor: CONSTANTINE, Mark, Poole, Dorset BH15 1AB (GB); CONSTANTINE, Margaret Joan, Poole, Dorset BH15 1AB (GB); AMBROSEN, Helen Elizabeth, Poole, Dorset BH15 1AB (GB); BIRD, Rowena Jacqueline, Poole, Dorset BH15 1AB (GB); CAMPBELL, Daniel James, Poole, Dorset BH15 1AB (GB)
(74) Representative: D Young & Co LLP

(57) **Abstract**

A cosmetic composition comprising
(i) water in an amount of from 5 to 30% by weight of the total composition;
(ii) saturated fats in an amount of 2% to 15% by weight of the total composition;
(iii) salt in an amount of 30% to 90% by weight of the total composition; and
(iv) an emulsifier,
wherein the cosmetic composition is an emulsion.

## Description

### FIELD OF THE INVENTION

The present invention relates to a product for use as a cosmetic, a process for producing said product, and a product prepared by the method.

### BACKGROUND TO THE INVENTION

The present invention relates to products particularly those for use in contact with the human body. The present invention relates to the increasing demand for fresh and natural cosmetics.

The rise of natural cosmetics to the pinnacle of the modern beauty industry is not a recent phenomenon. Humans have been using moisturisers since the Mesolithic era (10,000 years ago.) The Sumerians combined wine, tree oils and plant, animal or mineral materials before applying to the body. The ancient Egyptian civilisation is known to have used olive oil, sesame oil, myrrh resin as well as bitter almonds, honey and cardamom to care for the skin. But it was the celebrated Roman physician Galen who created cold cream in 200 BC, by melting beeswax into rose oil then adding water.

After centuries of homemade remedies, the use of cosmetics became wide spread in the in 1800's. The rise in popular new materials like Petroleum Jelly, Mineral Oil and Lanolin allowed the development of new cosmetics and high sales volumes. These new ingredients were far less expensive than beeswax and rose oil. Products such as Hinds Honey & Almond Cream formulated in 1872 by a Pharmacist from Maine in the U.S. enabled the use of cosmetics by many more people, particularly women anxious to keep their skin soft and healthy. However it was the advent of television and the rise in advertising that led to the initial boom in the 1930's.

Modern cosmetic products are formulated to have a very long shelf life. The shelf life of a typical product is thirty one months. However, before products reach a retail environment, they are shipped and then stored in warehouses, sometimes for years before sale. Products may be labelled with a 'best used by date' but without an indication of when they were made. Consequently many formulations are assembled with longevity more in mind than effect.

Consumers are increasingly concerned with the ingredients used in their cosmetics. There have been numerous reports in the media linking various materials in cosmetics with increased risk of various diseases including cancer. One materials category that has been associated with these stories on numerous occasions is preservatives.

The purpose of cosmetic preservatives is to prevent the growth of microorganisms (including yeasts & moulds), which would have a detrimental influence on the effect or the appearance of the product as well as being a risk to human health. However excessive use of preservatives can be employed to increase shelf life and therefore the profitability of cosmetics. In response to media or consumer pressure the industry may switch from one preservative system to another, however it would be more desirable to provide a range of effective preservative free cosmetics. By eliminating preservative systems from cosmetics, it would be possible to eliminate worries of consumers, such as bioaccumulation through repetitive use. It would also be possible to reduce the environmental impact of the cosmetic products. The use of preservatives in the manufacture of products and use by the consumer leads to the contamination of the environment by these materials.

The present invention seeks to provide a cosmetic product which does not contain preservatives at a significant concentration, if at all.

### SUMMARY OF THE INVENTION

In a first aspect, there is provided a cosmetic composition comprising
(i) water in an amount of from 5 to 70% by weight of the total composition;
wherein the cosmetic composition is free from preservatives, wherein free from preservatives is as defined herein.

In a second aspect, there is provided a process for the production of a cosmetic composition comprising
(a) water in an amount of from 5 to 70% by weight of the total composition;
the process comprising the step of mixing:
(i) water in an amount of from 5 to 70% by weight of the total composition; and
(ii) cosmetically acceptable ingredients
wherein the cosmetic composition is free from preservatives, wherein free from preservatives is as defined in the description.

In a third aspect, there is provided a product obtained or obtainable by a process for the production of a cosmetic composition comprising
(a) water in an amount of from 5 to 70% by weight of the total composition;
the process comprising the step of mixing:
(i) water in an amount of from 5 to 70% by weight of the total composition; and
(ii) cosmetically acceptable ingredients
wherein the cosmetic composition is free from preservatives, wherein free from preservatives is as defined in the description.

The term "free from preservatives" means that the composition contains any of the preservatives listed in Table 1 in amounts of no greater than the amounts specified. Preferably the composition is free from all of the preservatives listed in Table 1.

**Table 1**

| **Preservative** | **Present in an amount no greater than (based on the total composition)** |
|---|---|
| Benzoic acid | 0.062 % wt. |
| Sodium benzoate | 0.062 % wt. |
| Salts of benzoic acid excluding sodium benzoate | 0.003 % w/v |
| Propionic acid and salts thereof | 0.0032 % w/v |
| Salicylic acid and salts thereof | 1 % w/v |
| Hexa-2,4-dienoic acid and salts thereof | 0.4 % w/v |
| Formaldehyde | 0.01 % w/v |
| Paraformaldehyde | 0.01 % w/v |
| Biphenyl-2-ol and salts thereof | 1 x 10⁻⁵ % w/v |
| Pyrithione zinc | 1.6 x 10⁻⁴ % w/v |
| Inorganic sulphites | 1.6 x 10⁻⁵ % w/v |
| Hydrogen sulphites | 1.6 x 10⁻⁵ % w/v |
| Chlorobutanol | 0.25 % w/v |
| 4-Hydroxybenzoic acid and salts thereof and esters thereof | 1.2 x 10⁻² % w/v |
| 3-Acetyl-6-methylpyran-2,4(3H)-dione and salts thereof | 1 x 10⁻³ % w/v |
| Formic acid | 6 x 10⁻³ % w/v |
| Sodium formate | 6 x 10⁻³ % w/v |
| Methenamine 3-chloroallylochloride | 5 x 10⁻³ % w/v |
| 1-(4-Chlorophenoxy)-1-(imidazole-1-yl)-3,3-di methyl butan-2-one | 6.3 x 10⁻⁵ % w/v |
| 1,3-Bis (hydroxymethyl)-5,5-dimethylimidazolidine-2,4-dione | 6.3 x 10⁻⁵ % w/v |
| Benzyl alcohol | 0.0625 % w/v |
| 1-Hydroxy-4-methyl-6-(2,4,4-trimethylpentyl) 2-pyrindon | 1.6 x 10⁻⁵ % w/v |
| Monoethanolamine salt of 1-Hydroxy-4-methyl-6-(2,4,4-trimethylpentyl) 2-pyrindon | 1.6 x 10⁻⁵ % w/v |
| 2,2'-methylenebis(6-bromo-4-chlorophenol) | 2 x 10⁻³ % w/v |
| 4-Isopropyl-m-cresol | 0.1 % w/v |
| 5-Chloro-2-methyl-isothiazol-3(2H)-one | 1.6 x 10⁻³ % w/v |
| 2-methyl-isothiazol-3(2H)-one | 1.6 x 10⁻³ % w/v |
| 2-Benzyl-4-chlorophenol | 0.1 % w/v |
| 2-Chloroacetamide | 6.25 x 10⁻³ % w/v |
| N,N"-bis(chlorophenyl)-3,12-diamino-2,4,11,13-tetraazatetradecadiamidine | 5 x 10⁻⁴ % w/v |
| digluconate of N,N"-bis(chlorophenyl)-3,12-diamino-2,4, 11,13-tetraazatetradecadiamidine | 5 x 10⁻⁴ % w/v |
| diacetate of N,N"-bis(chlorophenyl)-3,12-diamino-2,4, 11,13-tetraazatetradecadiamidine | 5 x 10⁻⁴ % w/v |
| dihydrochloride of N,N"-bis(chlorophenyl)-3, 12-diamino-2,4, 11,13-tetraazatetradecadiamidine | 5 x 10⁻⁴ % w/v |
| 1-Phenoxypropanol-2-ol | 0.1 % w/v |
| Alkyl(C₁₂₋₂₂)trimethyl ammonium bromide | 6.1 x 10⁻³ % w/v |
| Alkyl(C₁₂₋₂₂)trimethyl ammonium chloride | 6.1 x 10⁻³ % w/v |
| 4,4-Dimethyl-1,3-oxazolidine | 3 x 10⁻⁶ % w/v |
| N-(hydroxymethyl)-N-(dihydroxymethyl-1,3-dioxo-2,5-imidazolidinyl-4)-N'(hydroxymethyl) urea | 3 x 10⁻⁵ % w/v |
| Benzenecarboximidamide, 4,4'-(1,6-hexanediylbis (oxy))bis- | 0.116 % w/v |
| Salts of Benzenecarboximidamide, 4,4'-(1,6-hexanediylbis (oxy))bis- | 0.116 % w/v |
| Glutaraldehyde (pentane-1,5-dial) | 5 x 10⁻⁴ % w/v |
| 5-Ethyl-3,7-dioxa-1-azabicyclo[3.3.0] octane | 1.5 x 10⁻⁴ % w/v |
| 3-(p-Chlorophenoxy)-propane-1,2 diol | 1.25 % w/v |
| Sodium hydroxymethylamino acetate | 2 x 10⁻⁵ % w/v |
| Silver chloride | 3.12 x 10⁻³ % w/v |
| Benzalkonium chloride | 4 x 10⁻³ % w/v |
| Benzalkonium bromide | 4 x 10⁻³ % w/v |
| Benzalkonium saccharinate | 4 x 10⁻³ % w/v |
| Phenylmethoxy methanol | 3 x 10⁻⁵ % w/v |
| 3-lodo-2-propynylbutylcarbamate | 6.25 x 10⁻³ % w/v |

In the process of the present invention, the process preferably excludes a step of addition one or more prohibited preservatives. The prohibited preservatives are those listed in Table 2.

**Table 2**

| | |
|---|---|
| Benzoic acid | 5-Chloro-2-methyl-isothiazol-3(2H)-one |
| Sodium benzoate | 2-methyl-isothiazol-3(2H)-one |
| Salts of benzoic acid excluding sodium benzoate | 2-Benzyl-4-chlorophenol |
| Propionic acid and salts thereof | 2-Chloroacetamide |
| Salicylic acid and salts thereof | N,N"-bis(chlorophenyl)-3,12-diamino-2,4,11,13-tetraazatetradecadiamidine |
| Hexa-2,4-dienoic acid and salts thereof | digluconate of N,N"-bis(chlorophenyl)-3,12-diamino-2,4, 11,13-tetraazatetradecadiamidine |
| Formaldehyde | diacetate of N,N"-bis(chlorophenyl)-3,12-diamino-2,4, 11,13-tetraazatetradecadiamidine |
| Paraformaldehyde | dihydrochloride of N,N"-bis(chlorophenyl)-3, 12-diamino-2,4,11,13-tetraazatetradecad iamidine |
| Biphenyl-2-ol and salts thereof | 1-Phenoxypropanol-2-ol |
| Pyrithione zinc | Alkyl(C₁₂₋₂₂)trimethyl ammonium bromide |
| Inorganic sulphites | Alkyl(C₁₂₋₂₂)trimethyl ammonium chloride |
| Hydrogen sulphites | 4,4-Dimethyl-1,3-oxazolidine |
| Chlorobutanol | N-(hydroxymethyl)-N-(dihydroxymethyl-1 ,3-dioxo-2,5-imidazolidinyl-4)-N'(hydroxymethyl) urea |
| 4-Hydroxybenzoic acid and salts thereof and esters thereof | Benzenecarboximidamide, 4,4'-(1,6-hexanediylbis (oxy))bis- |
| 3-Acetyl-6-methylpyran-2,4(3H)-dione and salts thereof | Salts of Benzenecarboximidamide, 4,4'-(1,6-hexanediylbis (oxy))bis- |
| Formic acid | Glutaraldehyde (pentane-1,5-dial) |
| Sodium formate | 5-Ethyl-3,7-dioxa-1-azabicyclo[3.3.0] octane |
| Methenamine 3-chloroallylochloride | 3-(p-Chlorophenoxy)-propane-1,2 diol |
| 1-(4-Chlorophenoxy)-1-(imidazole-1-yl)-3,3-di methyl butan-2-one | Sodium hydroxymethylamino acetate |
| 1,3-Bis (hydroxymethyl)-5,5-dimethylimidazolidine-2,4-dione | Silver chloride |
| Benzyl alcohol | Benzalkonium chloride |
| 1-Hydroxy-4-methyl-6-(2,4,4-trimethylpentyl) 2-pyrindon | Benzalkonium bromide |
| Monoethanolamine salt of 1-Hydroxy-4-methyl-6-(2,4,4-trimethylpentyl) 2-pyrindon | Benzalkonium saccharinate |
| 2,2'-methylenebis(6-bromo-4-chlorophenol) | Phenylmethoxy methanol |
| 4-Isopropyl-m-cresol | 3-lodo-2-propynylbutylcarbamate |

The present invention relates to a range cosmetics that are free of preservatives, yet are still resistant to microbial growth. They provide the user with a desirable product that is capable of being stored at room temperature and that may have a shelf life of from six weeks to twenty months. Typically the product will be packaged in a pot or bottle with a label noting when the product was made and advising when it should be used by.

For ease of reference, these and further aspects of the present invention are now discussed under appropriate section headings. However, the teachings under each section are not necessarily limited to each particular section.

### DETAILED DESCRIPTION

### Composition

As discussed herein, in one aspect of the present invention, there is provided a cosmetic composition comprising
(i) water in an amount of from 5 to 70% by weight of the total composition;
   wherein the cosmetic composition is free from preservatives, wherein free from preservatives is as defined herein.

The cosmetic composition may contain one or more additional components such as to provide the desired cosmetic composition.

In one aspect the cosmetic composition further comprises a saturated fat. By saturated fat it is meant a triglyceride in which each fatty acid chain is saturated. Preferably the cosmetic composition comprises saturated fats in an amount of 9% to 15% by weight of the total composition, such as in an amount of 9% to 11% by weight of the total composition.

Saturated fats, if present, may be provided from a number of sources of cosmetically acceptable fats. In one aspect, the saturated fats are provided by cocoa butter, olive oil, mango butter, coconut oil, Japan wax, avocado butter and mixtures thereof. In one aspect, the saturated fats are provided by cocoa butter, olive oil and mixtures thereof. Cocoa butter is particularly preferred. In one aspect the cosmetic composition comprises cocoa butter in an amount of at least 5% by weight of the total composition. In one aspect the cosmetic composition comprises cocoa butter in an amount of at least 6% by weight of the total composition. In a further aspect the cosmetic composition comprises cocoa butter in an amount of no greater than 15% by weight of the total composition.

Further, olive oil is a preferred source of saturated fat. In one aspect the cosmetic composition comprises olive oil in an amount of at least 25% by weight of the total composition. In a further aspect the cosmetic composition comprises olive oil in an amount of no greater than 50% by weight of the total composition

In a further aspect the cosmetic composition comprises
(i) water in an amount of from 14 to 18% by weight of the total composition;
(ii) saturated fats in an amount of 6.5% to 7.5% by weight of the total composition; and
(iii) salt in an amount of 45% to 55% by weight of the total composition.

In one aspect the cosmetic composition further comprises salt (i.e. sodium chloride). Preferably the cosmetic composition further comprises salt in an amount of 30% to 70% by weight of the total composition, such as in an amount of 40% to 60% by weight of the total composition or such as in an amount of 45% to 55% by weight of the total composition.

In one aspect the cosmetic composition further comprises an unsaturated fat. Preferably the cosmetic composition comprises unsaturated fats in an amount of 2% to 10% by weight of the total composition, such as in an amount of 3% to 7% by weight of the total composition.

In one aspect the cosmetic composition further comprises a clay. Preferably the cosmetic composition further comprises a clay such that the cosmetic composition contains a colloidal dispersion of the clay. The clay may be selected from all suitable clays. Preferably the clay is selected from kaolin, talc and mixtures thereof.

Preferably the clay is present in an amount of from 15 to 55% by weight of the total composition, such as in an amount of from 25 to 40% by weight of the total composition.

In one aspect the cosmetic composition further comprises a humectant. The humectant may be selected from all suitable humectants. Preferably the humectant is selected from honey, glycerine, monopropylene glycol, 1,2-propanediol, agave nectar, fruit syrups, herbal syrups, sugar solutions and mixtures thereof. More preferably the humectant is selected from honey, glycerine and mixtures thereof.

In one aspect the humectant is present in an amount of from 0.1% to 50% by weight of the total composition, such as in an amount of 30% to 50% by weight of the total composition, such as in an amount of 37% to 47% by weight of the total composition.

In one aspect the cosmetic composition comprises
(a) a colloidal dispersion of a clay;
(b) water in an amount of 10 to 30 % by weight of the total composition; and
(c) humectant in an amount of 25 to 60 % by weight of the total composition.

In one aspect the cosmetic composition comprises
(a) a colloidal dispersion of a clay wherein the clay is present in an amount of from 25 to 40% by weight of the total composition;
(b) water in an amount of 12 to 18 % by weight of the total composition; and
(c) humectant in an amount of 37 to 47 % by weight of the total composition.

In one aspect the cosmetic composition further comprises at least one additional component selected from binders, fillers, opacifiers, perfumes, colours, fragrances, scrubs, exfoliants and mixtures thereof. Preferably the cosmetic composition further comprises at least one additional component selected from colours, fragrances, scrubs, exfoliants and mixtures thereof.

In one aspect the cosmetic composition further comprises a fruit. In one aspect the cosmetic composition further comprises a fruit selected from lime, grapefruit and mixtures thereof.

In one aspect the cosmetic composition further comprises an emulsifier. The emulsifier may be selected from all suitable emulsifiers. Preferably the emulsifier is selected from triethanolamine, lactic acid, stearic acid, cetearyl alcohol, glyceryl stearate, cetyl alcohol, sodium laureth sulfate, glyceryl stearate, polyethylene glycol and mixtures thereof. In one preferred aspect the emulsifier is selected from stearic acid, cetearyl alcohol and mixtures thereof.

Preferably the cosmetic composition contains an emulsifier in an amount of from 1.5% to 15% by weight of the total composition.

In one aspect the cosmetic composition further comprises a protein. The emulsifier may be selected from all suitable proteins. Preferably the protein is selected tofu, banana, soya, soya lecithin, eggs and mixtures thereof.

Preferably the cosmetic composition contains a protein in an amount of from 1% to 30% by weight of the total composition.

In one aspect the cosmetic composition is a liquid composition. In one aspect the cosmetic composition is a solid composition. Solid products of the present invention are compositions which can substantially sustain their physical shape when unsupported by external means, e.g. packaging etc. Thus, they are considered to be solid, solid like, in solid form or in solid-like form at room temperature. For the avoidance of doubt the solid product must remain substantially solid at up to 30°C.

By solid-like, it is understood that some materials are considered on a day to day basis to be solid, yet over an extremely long period of time, may alter in shape, e.g. amorphous materials such as glass etc. However, they are considered to be solid-like as, for the purpose they fulfil, they are solid. The solid form of the solid compositions of the present invention mean that external packaging is not required to maintain the shape of the composition.

### Water

As discussed herein, in one aspect of the present invention, there is provided a cosmetic composition comprising (i) water in an amount of from 5 to 70% by weight of the total composition;

In various aspects of the present invention water is present in an amount of
- from 5 to 50% by weight of the total composition;
- from 10 to 50% by weight of the total composition
- from 25 to 45% by weight of the total composition
- from 30 to 40% by weight of the total composition
- from 32 to 38% by weight of the total composition
- from 10 to 25% by weight of the total composition.
- from 10 to 20% by weight of the total composition
- from 12 to 20% by weight of the total composition
- 12 to 18% by weight of the total composition.
- from 14 to 18% by weight of the total composition

### Cream, Moisturiser or Lotion (Emulsion)

In one preferred aspect, the cosmetic composition is a cream or moisturiser or lotion.

In one preferred aspect the cosmetic composition (such as a cream or moisturiser or lotion) comprises
(i) water in an amount of from 20 to 70% by weight of the total composition;
(ii) saturated fats in an amount of 6% to 15% by weight of the total composition.

In one preferred aspect the cosmetic composition (such as a cream or moisturiser or lotion) comprises
(i) water in an amount of from 20 to 70% by weight of the total composition;
(ii) saturated fats in an amount of 9% to 15% by weight of the total composition.

In one preferred aspect the cosmetic composition (such as a cream or moisturiser or lotion) comprises
(i) water in an amount of from 30 to 70% by weight of the total composition;
(ii) saturated fats in an amount of 6% to 15% by weight of the total composition.

In one preferred aspect the cosmetic composition (such as a cream or moisturiser or lotion) comprises
(i) water in an amount of from 30 to 70% by weight of the total composition;
(ii) saturated fats in an amount of 9% to 15% by weight of the total composition.

In one preferred aspect the cosmetic composition (such as a cream or moisturiser or lotion) comprises
(i) water in an amount of from 33 to 70% by weight of the total composition;
(ii) saturated fats in an amount of 6% to 15% by weight of the total composition.

In one preferred aspect the cosmetic composition (such as a cream or moisturiser or lotion) comprises
(i) water in an amount of from 33 to 70% by weight of the total composition;
(ii) saturated fats in an amount of 9% to 15% by weight of the total composition.

In one preferred aspect the cosmetic composition (such as a cream or moisturiser or lotion) comprises
(i) water in an amount of from 20 to 45% by weight of the total composition;
(ii) saturated fats in an amount of 6% to 15% by weight of the total composition.

In one preferred aspect the cosmetic composition (such as a cream or moisturiser or lotion) comprises
(i) water in an amount of from 20 to 45% by weight of the total composition;
(ii) saturated fats in an amount of 9% to 15% by weight of the total composition.

Preferably water is present in an amount of from 25 to 70% by weight of the total composition (such as a cream or moisturiser or lotion), such as from 30 to 70% by weight of the total composition, such as from 33 to 70% by weight of the total composition, such as from 25 to 45% by weight of the total composition, such as from 30 to 40% by weight of the total composition, such as from 32 to 38% by weight of the total composition.

Preferably the cosmetic composition (such as a cream or moisturiser or lotion) comprises saturated fats present in an amount of 6% to 13% by weight of the total composition, such as in an amount of 6% to 11% by weight of the total composition, such as in an amount of 9% to 13% by weight of the total composition, such as in an amount of 9% to 11% by weight of the total composition.

Preferably the cosmetic composition (such as a cream or moisturiser or lotion) comprises
(i) water in an amount of from 32 to 38% by weight of the total composition;
(ii) saturated fats in an amount of 6% to 11% by weight of the total composition.

Preferably the cosmetic composition (such as a cream or moisturiser or lotion) comprises
(i) water in an amount of from 32 to 38% by weight of the total composition;
(ii) saturated fats in an amount of 9% to 11% by weight of the total composition.

Preferably the cream or moisturiser comprises cocoa butter in an amount of at least 6% by weight of the total composition. Preferably the cream or moisturiser comprises cocoa butter in an amount of at least 8% by weight of the total composition. Preferably the cream or moisturiser comprises cocoa butter in an amount of no greater than 15% by weight of the total composition. Preferably the cream or moisturiser comprises olive oil in an amount of at least 25% by weight of the total composition. Preferably the cream or moisturiser comprises olive oil in an amount of at least 35% by weight of the total composition. Preferably the cream or moisturiser comprises olive oil in an amount of no greater than 50% by weight of the total composition

We have found that by preparing a formulation within certain parameters a cosmetic emulsion may be prepared which does not require the presence of preservatives (as described herein). Thus in a further broad aspect the present invention provides a cosmetic composition comprising
(i) water in an amount of from 30 to 70% by weight of the total composition;
(ii) saturated fats in an amount of 6% to 15% by weight of the total composition.
In a further broad aspect the present invention provides a cosmetic composition comprising
(i) water in an amount of from 30 to 70% by weight of the total composition;
(ii) saturated fats in an amount of 9% to 15% by weight of the total composition.
In a further broad aspect the present invention provides a cosmetic composition comprising
(i) water in an amount of from 20 to 45% by weight of the total composition;
(ii) saturated fats in an amount of 6% to 15% by weight of the total composition.
In a further broad aspect the present invention provides a cosmetic composition comprising
(i) water in an amount of from 20 to 45% by weight of the total composition;
(ii) saturated fats in an amount of 9% to 15% by weight of the total composition.

In a further aspect the present invention provides a cosmetic emulsion composition comprising
(i) water in an amount of from 20 to 70% by weight of the total composition;
(ii) saturated fats in an amount of 6 to 15% (preferably 9 to 15%) by weight of the total composition. This system is referred to as the 'emulsion of the invention'. It is understood that by producing such a formulation an emulsion is formed which is 'tight'. It is understood that the dispersed phase is bound such that micro-organisms can not easily access both the continuous phase and dispersed phase. Access to both phases is typically required for growth of micro-organisms which require both access to water and access to oil as a substrate for growth. Thus a system is provided which is considered to be "self preserving" - growth of microorganisms is inhibited without the use of an additional preservative. Therefore the emulsion may be free from preservatives, wherein free from preservatives is as defined herein.

In one preferred aspect the cosmetic emulsion composition comprises
(i) water in an amount of from 30 to 70% by weight of the total composition;
(ii) saturated fats in an amount of 6% to 15% by weight of the total composition.

In one preferred aspect the cosmetic emulsion composition comprises
(i) water in an amount of from 30 to 70% by weight of the total composition;
(ii) saturated fats in an amount of 9% to 15% by weight of the total composition.

In one preferred aspect the cosmetic emulsion composition comprises
(i) water in an amount of from 33 to 70% by weight of the total composition;
(ii) saturated fats in an amount of 6% to 15% by weight of the total composition.

In one preferred aspect the cosmetic emulsion composition comprises
(i) water in an amount of from 33 to 70% by weight of the total composition;
(ii) saturated fats in an amount of 9% to 15% by weight of the total composition.

In one preferred aspect the cosmetic emulsion composition comprises
(i) water in an amount of from 20 to 45% by weight of the total composition;
(ii) saturated fats in an amount of 6% to 15% by weight of the total composition.

In one preferred aspect the cosmetic emulsion composition comprises
(i) water in an amount of from 20 to 45% by weight of the total composition;
(ii) saturated fats in an amount of 9% to 15% by weight of the total composition.

The emulsion may be a water-in-oil emulsion or an oil-in-water emulsion. In one preferred aspect the emulsion is an oil-in-water emulsion.

Preferably water is present in the emulsion of the invention in an amount of from 25 to 70% by weight of the total composition, such as from 30 to 70% by weight of the total composition, such as from 33 to 70% by weight of the total composition, such as from 25 to 45% by weight of the total composition, such as from 30 to 40% by weight of the total composition, such as from 32 to 38% by weight of the total composition.

Preferably the emulsion of the invention (such as a cream or moisturiser or lotion) comprises saturated fats present in an amount of 6% to 13% by weight of the total composition, such as in an amount of 6% to 11% by weight of the total composition, such as in an amount of 9% to 13% by weight of the total composition, such as in an amount of 9% to 11% by weight of the total composition.

Preferably the emulsion of the invention (such as a cream or moisturiser or lotion) comprises
(i) water in an amount of from 32 to 38% by weight of the total composition;
(ii) saturated fats in an amount of 6% to 11% by weight of the total composition.

Preferably the emulsion of the invention (such as a cream or moisturiser or lotion) comprises
(i) water in an amount of from 32 to 38% by weight of the total composition;
(ii) saturated fats in an amount of 9% to 11% by weight of the total composition.

Preferably the emulsion of the invention comprises cocoa butter. Preferably the emulsion of the invention comprises cocoa butter in an amount of at least 6% by weight of the total composition. Preferably the emulsion of the invention comprises cocoa butter in an amount of at least 8% by weight of the total composition. Preferably the emulsion of the invention comprises cocoa butter in an amount of no greater than 15% by weight of the total composition. Preferably the emulsion of the invention comprises olive oil in an amount of at least 25% by weight of the total composition. Preferably the emulsion of the invention comprises olive oil in an amount of at least 35% by weight of the total composition. Preferably the emulsion of the invention comprises olive oil in an amount of no greater than 50% by weight of the total composition

Preferably the emulsion of the invention comprises an emulsifier. The emulsifier may be present in any amount required to provide the desired emulsion. Preferably the emulsifier is present in an amount of at least 0.1% by weight of the total composition. Preferably the emulsifier is present in an amount of at least 0.2% by weight of the total composition. Preferably the emulsifier is present in an amount of at least 0.5% by weight of the total composition. Preferably the emulsifier is present in an amount of at least 1% by weight of the total composition. Preferably the emulsifier is present in an amount of at least 2% by weight of the total composition. Preferably the emulsifier is present in an amount of at least 2.5% by weight of the total composition. Preferably the emulsifier is present in an amount of at least 3% by weight of the total composition. Preferably the emulsifier is present in an amount of at least 3.5% by weight of the total composition. Preferably the emulsifier is present in an amount of at least 4% by weight of the total composition. Preferably the emulsifier is present in an amount of at least 5% by weight of the total composition. Preferably the emulsifier is present in an amount of at least 5.3% by weight of the total composition.

Preferably the emulsifier is present in an amount of no greater than 15% by weight of the total composition. Preferably the emulsifier is present in an amount of no greater than 12% by weight of the total composition. Preferably the emulsifier is present in an amount of no greater than 10% by weight of the total composition. Preferably the emulsifier is present in an amount of no greater than 9% by weight of the total composition. Preferably the emulsifier is present in an amount of no greater than 8% by weight of the total composition. Preferably the emulsifier is present in an amount of no greater than 7% by weight of the total composition. Preferably the emulsifier is present in an amount of no greater than 6% by weight of the total composition. Preferably the emulsifier is present in an amount of no greater than 5.3% by weight of the total composition.

Preferably the emulsion of the invention comprises an emulsifier. The emulsifier may be present in any amount required to provide the desired emulsion. Preferably the emulsifier is present in an amount of 0.1-15% by weight of the total composition. Preferably the emulsifier is present in an amount of 0.2-12% by weight of the total composition. Preferably the emulsifier is present in an amount of 0.5-10% by weight of the total composition. Preferably the emulsifier is present in an amount of 1-9% by weight of the total composition. Preferably the emulsifier is present in an amount of 2-8% by weight of the total composition. Preferably the emulsifier is present in an amount of 2.5-7% by weight of the total composition. Preferably the emulsifier is present in an amount of 3-6% by weight of the total composition. Preferably the emulsifier is present in an amount of 3-5.5% by weight of the total composition. Preferably the emulsifier is present in an amount of 3.5-5.5% by weight of the total composition. Preferably the emulsifier is present in an amount of 4-5.5% by weight of the total composition.

The emulsifier may be selected from suitable emulsifiers. The emulsifier may be selected from triethanolamine, lactic acid, stearic acid, cetearyl alcohol, glyceryl stearate, cetyl alcohol, sodium laureth sulfate, glyceryl stearate and polyethylene glycol. One preferred emulsifier is cetearyl alcohol. Cetearyl alcohol (also known as cetostearyl alcohol or cetylstearyl alcohol) is a mixture of fatty alcohols, consisting predominantly of cetyl and stearyl alcohols. One preferred emulsifier is stearic acid. Preferably the emulsifier is selected from cetearyl alcohol, stearic acid and mixtures thereof.

Preferably the emulsion of the invention comprises a nut oil. Preferably the nut oil is almond oil. Nut oils, such as almond oil, are able to reduce the viscosity of the cosmetic composition and also beneficial to the skin when applied by a user. The emulsion of the invention may comprise nut oil (such as almond oil) in an amount of at least 1% by weight of the total composition. Preferably the emulsion of the invention comprises nut oil (such as almond oil) in an amount of at least 5% by weight of the total composition. Preferably the emulsion of the invention comprises nut oil (such as almond oil) in an amount of at least 9% by weight of the total composition. Preferably the emulsion of the invention comprises nut oil (such as almond oil) in an amount of no greater than 70% by weight of the total composition. Preferably the emulsion of the invention comprises nut oil (such as almond oil) in an amount of no greater than 60% by weight of the total composition. Preferably the emulsion of the invention comprises nut oil (such as almond oil) in an amount of no greater than 50% by weight of the total composition. Preferably the emulsion of the invention comprises nut oil (such as almond oil) in an amount of 5-70% by weight of the total composition. Preferably the emulsion of the invention comprises nut oil (such as almond oil) in an amount of 5-60% by weight of the total composition. Preferably the emulsion of the invention comprises nut oil (such as almond oil) in an amount of 5-50% by weight of the total composition. Preferably the emulsion of the invention comprises nut oil (such as almond oil) in an amount of 9-41% by weight of the total composition.

Preferably the emulsion of the invention comprises glycerine. The glycerine may act as a humectant which reduces the level of active water and thereby inhibits the growth of microorganisms. The emulsion of the invention may comprise glycerine in an amount of at least 1% by weight of the total composition. Preferably the emulsion of the invention comprises glycerine in an amount of at least 5% by weight of the total composition. Preferably the emulsion of the invention comprises glycerine in an amount of at least 7% by weight of the total composition. Preferably the emulsion of the invention comprises glycerine in an amount of no greater than 30% by weight of the total composition. Preferably the emulsion of the invention comprises glycerine in an amount of no greater than 20% by weight of the total composition. Preferably the emulsion of the invention comprises glycerine in an amount of no greater than 15% by weight of the total composition. Preferably the emulsion of the invention comprises glycerine in an amount of no greater than 10% by weight of the total composition. Preferably the emulsion of the invention comprises glycerine in an amount of 1-30% by weight of the total composition. Preferably the emulsion of the invention comprises glycerine in an amount of 5-20% by weight of the total composition. Preferably the emulsion of the invention comprises glycerine in an amount of 7-15% by weight of the total composition. Preferably the emulsion of the invention comprises glycerine in an amount of 7-10% by weight of the total composition.

The saturated fats may be provided from a number of sources of cosmetically acceptable fats. In one aspect, the saturated fats are provided by cocoa butter, olive oil, mango butter, coconut oil, Japan wax, avocado butter and mixtures thereof. In one aspect, the saturated fats are provided by cocoa butter, olive oil and mixtures thereof. Cocoa butter is particularly preferred. In one aspect the emulsion composition comprises cocoa butter in an amount of at least 6% by weight of the total composition. In a further aspect the emulsion composition comprises cocoa butter in an amount of no greater than 15% by weight of the total composition.

Each of the above preferred aspects of the present invention may be combined to provide a preferred emulsion of the invention.

In a preferred aspect the present invention provides a cosmetic emulsion composition comprising
(i) water in an amount of from 33 to 70% by weight of the total composition;
(ii) saturated fats in an amount of 9% to 15% by weight of the total composition;
(iii) glycerine in an amount of 7% to 10% by weight of the total composition;
(iv) almond oil in an amount of 9% to 41% by weight of the total composition; and
(v) emulsifier (preferably cetearyl alcohol) in an amount of 1.7% to 1% by weight of the total composition.

### Scrub

In one preferred aspect, the cosmetic composition is a scrub or facial wash.

Preferably the cosmetic composition (such as a scrub) of the present invention comprises salt in an amount of at least 10% by weight of the total composition. By salt it is meant sodium chloride. Preferably the cosmetic composition (such as a scrub) of the present invention comprises salt in an amount of at least 20% by weight of the total composition. Preferably the cosmetic composition (such as a scrub) of the present invention comprises salt in an amount of at least 30% by weight of the total composition. Preferably the cosmetic composition (such as a scrub) of the present invention comprises salt in an amount of at least 40% by weight of the total composition. Preferably the cosmetic composition (such as a scrub) of the present invention comprises salt in an amount of at least 50% by weight of the total composition.

Preferably the cosmetic composition (such as a scrub) of the present invention comprises salt in an amount of no greater than 90% by weight of the total composition. Preferably the cosmetic composition (such as a scrub) of the present invention comprises salt in an amount of no greater than 80% by weight of the total composition. Preferably the cosmetic composition (such as a scrub) of the present invention comprises salt in an amount of no greater than 70% by weight of the total composition. Preferably the cosmetic composition (such as a scrub) of the present invention comprises salt in an amount of no greater than 60% by weight of the total composition. Preferably the cosmetic composition (such as a scrub) of the present invention comprises salt in an amount of no greater than 50% by weight of the total composition.

Preferably the cosmetic composition (such as a scrub) of the present invention comprises salt in an amount of 10% to 90% by weight of the total composition. Preferably the cosmetic composition (such as a scrub) of the present invention comprises salt in an amount of 20% to 80% by weight of the total composition. Preferably the cosmetic composition (such as a scrub) of the present invention comprises salt in an amount of 30% to 70% by weight of the total composition. Preferably the cosmetic composition (such as a scrub) of the present invention comprises salt in an amount of 40% to 60% by weight of the total composition.

Preferably the cosmetic composition (such as a scrub) comprises salt in an amount of 40% to 60% by weight of the total composition, such as in an amount of 45% to 55% by weight of the total composition.

The cosmetic composition (such as a scrub containing salt in an amount of 30 to 70% by weight of the total composition or other amount described herein) comprises water in an amount of at least 5% by weight of the total composition. Preferably the cosmetic composition (such as a scrub containing salt in an amount of 30 to 70% by weight of the total composition or other amount described herein) comprises water in an amount of at least 6% by weight of the total composition. Preferably the cosmetic composition (such as a scrub containing salt in an amount of 30 to 70% by weight of the total composition or other amount described herein) comprises water in an amount of at least 7% by weight of the total composition.

Preferably the cosmetic composition (such as a scrub containing salt in an amount of 30 to 70% by weight of the total composition or other amount described herein) comprises water in an amount of no greater than 30% by weight of the total composition. Preferably the cosmetic composition (such as a scrub containing salt in an amount of 30 to 70% by weight of the total composition or other amount described herein) comprises water in an amount of no greater than 25% by weight of the total composition. Preferably the cosmetic composition (such as a scrub containing salt in an amount of 30 to 70% by weight of the total composition or other amount described herein) comprises water in an amount of no greater than 20% by weight of the total composition. Preferably the cosmetic composition (such as a scrub containing salt in an amount of 30 to 70% by weight of the total composition or other amount described herein) comprises water in an amount of no greater than 15% by weight of the total composition. Preferably the cosmetic composition (such as a scrub containing salt in an amount of 30 to 70% by weight of the total composition or other amount described herein) comprises water in an amount of no greater than 10% by weight of the total composition. Preferably the cosmetic composition (such as a scrub containing salt in an amount of 30 to 70% by weight of the total composition or other amount described herein) comprises water in an amount of no greater than 9% by weight of the total composition. Preferably the cosmetic composition (such as a scrub containing salt in an amount of 30 to 70% by weight of the total composition or other amount described herein) comprises water in an amount of no greater than 8% by weight of the total composition. Preferably the cosmetic composition (such as a scrub containing salt in an amount of 30 to 70% by weight of the total composition or other amount described herein) comprises water in an amount of no greater than 7% by weight of the total composition.

Preferably the cosmetic composition (such as a scrub containing salt in an amount of 30 to 70% by weight of the total composition or other amount described herein) comprises water in an amount of from 5 to 30% by weight of the total composition. Preferably the cosmetic composition (such as a scrub containing salt in an amount of 30 to 70% by weight of the total composition or other amount described herein) comprises water in an amount of from 5 to 25% by weight of the total composition. Preferably the cosmetic composition (such as a scrub containing salt in an amount of 30 to 70% by weight of the total composition or other amount described herein) comprises water in an amount of from 5 to 20% by weight of the total composition. Preferably the cosmetic composition (such as a scrub containing salt in an amount of 30 to 70% by weight of the total composition or other amount described herein) comprises water in an amount of from 5 to 15% by weight of the total composition. Preferably the cosmetic composition (such as a scrub containing salt in an amount of 30 to 70% by weight of the total composition or other amount described herein) comprises water in an amount of from 5 to 12% by weight of the total composition. Preferably the cosmetic composition (such as a scrub containing salt in an amount of 30 to 70% by weight of the total composition or other amount described herein) comprises water in an amount of from 5 to 10% by weight of the total composition. Preferably the cosmetic composition (such as a scrub containing salt in an amount of 30 to 70% by weight of the total composition or other amount described herein) comprises water in an amount of from 5 to 9% by weight of the total composition. Preferably the cosmetic composition (such as a scrub containing salt in an amount of 30 to 70% by weight of the total composition or other amount described herein) comprises water in an amount of from 6 to 8% by weight of the total composition.

Preferably the cosmetic composition (such as a scrub) comprises water in an amount of from 10 to 20% by weight of the total composition, such as in an amount of from 12 to 20% by weight of the total composition, such as in an amount of from 14 to 18% by weight of the total composition.

Preferably the cosmetic composition (such as a scrub containing salt in an amount of 30 to 70% by weight of the total composition or other amount described herein) comprises saturated fats in an amount of at least 2% by weight of the total composition. Preferably the cosmetic composition (such as a scrub containing salt in an amount of 30 to 70% by weight of the total composition or other amount described herein) comprises saturated fats in an amount of at least 3% by weight of the total composition. Preferably the cosmetic composition (such as a scrub containing salt in an amount of 30 to 70% by weight of the total composition or other amount described herein) comprises saturated fats in an amount of at least 4% by weight of the total composition. Preferably the cosmetic composition (such as a scrub containing salt in an amount of 30 to 70% by weight of the total composition or other amount described herein) comprises saturated fats in an amount of at least 5% by weight of the total composition. Preferably the cosmetic composition (such as a scrub containing salt in an amount of 30 to 70% by weight of the total composition or other amount described herein) comprises saturated fats in an amount of at least 6% by weight of the total composition.

Preferably the cosmetic composition (such as a scrub containing salt in an amount of 30 to 70% by weight of the total composition or other amount described herein) comprises saturated fats in an amount of no greater than 25% by weight of the total composition. Preferably the cosmetic composition (such as a scrub containing salt in an amount of 30 to 70% by weight of the total composition or other amount described herein) comprises saturated fats in an amount of no greater than 20% by weight of the total composition. Preferably the cosmetic composition (such as a scrub containing salt in an amount of 30 to 70% by weight of the total composition or other amount described herein) comprises saturated fats in an amount of no greater than 15% by weight of the total composition. Preferably the cosmetic composition (such as a scrub containing salt in an amount of 30 to 70% by weight of the total composition or other amount described herein) comprises saturated fats in an amount of no greater than 10% by weight of the total composition. Preferably the cosmetic composition (such as a scrub containing salt in an amount of 30 to 70% by weight of the total composition or other amount described herein) comprises saturated fats in an amount of no greater than 8% by weight of the total composition.

Preferably the cosmetic composition (such as a scrub containing salt in an amount of 30 to 70% by weight of the total composition or other amount described herein) comprises saturated fats in an amount of 2% to 25% by weight of the total composition. Preferably the cosmetic composition (such as a scrub containing salt in an amount of 30 to 70% by weight of the total composition or other amount described herein) comprises saturated fats in an amount of 2% to 20% by weight of the total composition. Preferably the cosmetic composition (such as a scrub containing salt in an amount of 30 to 70% by weight of the total composition or other amount described herein) comprises saturated fats in an amount of 2% to 15% by weight of the total composition. Preferably the cosmetic composition (such as a scrub containing salt in an amount of 30 to 70% by weight of the total composition or other amount described herein) comprises saturated fats in an amount of 2% to 10% by weight of the total composition. Preferably the cosmetic composition (such as a scrub containing salt in an amount of 30 to 70% by weight of the total composition or other amount described herein) comprises saturated fats in an amount of 2% to 8% by weight of the total composition. Preferably the cosmetic composition (such as a scrub containing salt in an amount of 30 to 70% by weight of the total composition or other amount described herein) comprises saturated fats in an amount of 3% to 8% by weight of the total composition. Preferably the cosmetic composition (such as a scrub containing salt in an amount of 30 to 70% by weight of the total composition or other amount described herein) comprises saturated fats in an amount of 4% to 8% by weight of the total composition. Preferably the cosmetic composition (such as a scrub containing salt in an amount of 30 to 70% by weight of the total composition or other amount described herein) comprises saturated fats in an amount of 5% to 8% by weight of the total composition. Preferably the cosmetic composition (such as a scrub containing salt in an amount of 30 to 70% by weight of the total composition or other amount described herein) comprises saturated fats in an amount of 6% to 8% by weight of the total composition.

Preferably the cosmetic composition (such as a scrub) comprises saturated fats in an amount of 6.5% to 7.5% by weight of the total composition, such as in an amount of approximately 7% by weight of the total composition.

Preferably the cosmetic composition (such as a scrub) comprises
(i) water in an amount of from 5 to 30% by weight of the total composition;
(ii) saturated fats in an amount of 2% to 15% by weight of the total composition; and
(iii) salt in an amount of 30% to 70% by weight of the total composition.

Preferably the cosmetic composition (such as a scrub) comprises
(i) water in an amount of from 5 to 25% by weight of the total composition;
(ii) saturated fats in an amount of 2% to 12% by weight of the total composition; and
(iii) salt in an amount of 30% to 70% by weight of the total composition.

Preferably the cosmetic composition (such as a scrub) comprises
(i) water in an amount of from 5 to 25% by weight of the total composition;
(ii) saturated fats in an amount of 2% to 25% by weight of the total composition; and
(iii) salt in an amount of 30% to 70% by weight of the total composition.

Preferably the cosmetic composition (such as a scrub) comprises
(i) water in an amount of from 5 to 25% by weight of the total composition;
(ii) saturated fats in an amount of 2% to 8% by weight of the total composition; and
(iii) salt in an amount of 30% to 70% by weight of the total composition.

Preferably the cosmetic composition (such as a scrub) comprises
(i) water in an amount of from 14 to 18% by weight of the total composition;
(ii) saturated fats in an amount of 6.5% to 7.5% by weight of the total composition; and
(iii) salt in an amount of 45% to 55% by weight of the total composition.

We have found that by preparing a formulation within certain parameters a cosmetic composition may be prepared which does not require the presence of preservatives (as described herein). Thus in a further broad aspect the present invention provides a cosmetic composition comprising
(i) water in an amount of from 5 to 30% by weight of the total composition;
(ii) saturated fats in an amount of 2% to 15% by weight of the total composition; and
(iii) salt in an amount of 30% to 90% by weight of the total composition.

This system is referred to as the 'salt composition of the invention'. It is understood that by producing such a formulation a cosmetic composition is formed which inhibits the growth of microorganisms. The high salt content of the solid composition of the invention itself inhibits the growth of microorganisms. Furthermore the high salt content reduces the active water available from microorganism growth. Cosmetic systems containing low active water are known in the prior art. For example, soaps typically have no water content and do not allow microorganism growth. However, such soap compositions cannot be left on the skin for any prolonged period without causing irritation to the skin. Thus a system is provided which is considered to be "self preserving" - growth of microorganisms is inhibited without the use of an additional preservative. Therefore, the salt composition may be free from preservatives, wherein free from preservatives is as defined herein.

In a further broad aspect the present invention provides a cosmetic composition comprising
(i) water in an amount of from 5 to 25% by weight of the total composition;
(ii) saturated fats in an amount of 2 to 15% by weight of the total composition; and
(iii) salt in an amount of 30 to 80% by weight of the total composition.

In a further broad aspect the present invention provides a cosmetic composition comprising
(i) water in an amount of from 5 to 25% by weight of the total composition;
(ii) saturated fats in an amount of 2 to 15% by weight of the total composition; and
(iii) salt in an amount of 30 to 70% by weight of the total composition.

In a further broad aspect the present invention provides a cosmetic composition comprising
(i) water in an amount of from 5 to 25% by weight of the total composition;
(ii) saturated fats in an amount of 2 to 8% by weight of the total composition; and
(iii) salt in an amount of 30 to 70% by weight of the total composition.

Preferably the salt composition of the invention comprises water in an amount of from 5 to 30% by weight of the total composition. Preferably the salt composition of the invention comprises water in an amount of from 5 to 25% by weight of the total composition. Preferably the salt composition of the invention comprises water in an amount of from 5 to 20% by weight of the total composition. Preferably the salt composition of the invention comprises water in an amount of from 5 to 15% by weight of the total composition. Preferably the salt composition of the invention comprises water in an amount of from 5 to 12% by weight of the total composition. Preferably the salt composition of the invention comprises water in an amount of from 5 to 10% by weight of the total composition. Preferably the salt composition of the invention comprises water in an amount of from 5 to 9% by weight of the total composition. Preferably the salt composition of the invention comprises water in an amount of from 6 to 8% by weight of the total composition.

Preferably the salt composition of the invention comprises saturated fats in an amount of 2% to 25% by weight of the total composition. Preferably the salt composition of the invention comprises saturated fats in an amount of 2% to 20% by weight of the total composition. Preferably the salt composition of the invention comprises saturated fats in an amount of 2% to 15% by weight of the total composition. Preferably the salt composition of the invention comprises saturated fats in an amount of 2% to 10% by weight of the total composition. Preferably the salt composition of the invention comprises saturated fats in an amount of 2% to 8% by weight of the total composition. Preferably the salt composition of the invention comprises saturated fats in an amount of 3% to 8% by weight of the total composition. Preferably the salt composition of the invention comprises saturated fats in an amount of 4% to 8% by weight of the total composition. Preferably the salt composition of the invention comprises saturated fats in an amount of 5% to 8% by weight of the total composition. Preferably the salt composition of the invention comprises saturated fats in an amount of 6% to 8% by weight of the total composition.

Preferably the salt composition of the invention comprises salt in an amount of 10% to 90% by weight of the total composition. Preferably the salt composition of the invention comprises salt in an amount of 20% to 80% by weight of the total composition. Preferably the salt composition of the invention comprises salt in an amount of 30% to 70% by weight of the total composition. Preferably the salt composition of the invention comprises salt in an amount of 40% to 60% by weight of the total composition.

Preferably the salt composition of the invention comprises an emulsifier. The emulsifier may be present in any amount required to provide the desired emulsion. Preferably the emulsifier is present in an amount of at least 1% by weight of the total composition. Preferably the emulsifier is present in an amount of at least 2% by weight of the total composition. Preferably the emulsifier is present in an amount of at least 3% by weight of the total composition. Preferably the emulsifier is present in an amount of at least 4% by weight of the total composition. Preferably the emulsifier is present in an amount of at least 5% by weight of the total composition. Preferably the emulsifier is present in an amount of at least 6% by weight of the total composition. Preferably the emulsifier is present in an amount of at least 7% by weight of the total composition. Preferably the emulsifier is present in an amount of at least 8% by weight of the total composition. Preferably the emulsifier is present in an amount of at least 9% by weight of the total composition.

Preferably the emulsifier is present in an amount of no greater than 20% by weight of the total composition. Preferably the emulsifier is present in an amount of no greater than 19% by weight of the total composition. Preferably the emulsifier is present in an amount of no greater than 18% by weight of the total composition. Preferably the emulsifier is present in an amount of no greater than 17% by weight of the total composition. Preferably the emulsifier is present in an amount of no greater than 16% by weight of the total composition. Preferably the emulsifier is present in an amount of no greater than 15% by weight of the total composition. Preferably the emulsifier is present in an amount of no greater than 14% by weight of the total composition. Preferably the emulsifier is present in an amount of no greater than 13% by weight of the total composition. Preferably the emulsifier is present in an amount of no greater than 12% by weight of the total composition. Preferably the emulsifier is present in an amount of no greater than 11% by weight of the total composition. Preferably the emulsifier is present in an amount of no greater than 10% by weight of the total composition.

Preferably the salt composition of the invention comprises an emulsifier. The emulsifier may be present in any amount required to provide the desired emulsion. Preferably the emulsifier is present in an amount of 1-20% by weight of the total composition. Preferably the emulsifier is present in an amount of at least 2-19% by weight of the total composition. Preferably the emulsifier is present in an amount of at least 3-18% by weight of the total composition. Preferably the emulsifier is present in an amount of at least 4-16% by weight of the total composition. Preferably the emulsifier is present in an amount of at least 5-15% by weight of the total composition. Preferably the emulsifier is present in an amount of at least 6-14% by weight of the total composition. Preferably the emulsifier is present in an amount of at least 7-13% by weight of the total composition. Preferably the emulsifier is present in an amount of at least 7-12% by weight of the total composition. Preferably the emulsifier is present in an amount of at least 7-11% by weight of the total composition. Preferably the emulsifier is present in an amount of at least 8-11% by weight of the total composition. Preferably the emulsifier is present in an amount of at least 8-10% by weight of the total composition. Preferably the emulsifier is present in an amount of at least 9-10% by weight of the total composition.

The emulsifier may be selected from suitable emulsifiers. The emulsifier may be selected from triethanolamine, lactic acid, stearic acid, cetearyl alcohol, glyceryl stearate, cetyl alcohol, sodium laureth sulfate, glyceryl stearate and polyethylene glycol. One preferred emulsifier is cetearyl alcohol. Cetearyl alcohol (also known as cetostearyl alcohol or cetylstearyl alcohol) is a mixture of fatty alcohols, consisting predominantly of cetyl and stearyl alcohols. One preferred emulsifier is stearic acid. Preferably the emulsifier is selected from cetearyl alcohol, stearic acid and mixtures thereof.

Preferably the salt composition of the invention comprises glycerine. The glycerine may act as a humectant which reduces the level of active water and thereby inhibits the growth of microorganisms. The salt composition of the invention may comprise glycerine in an amount of at least 1% by weight of the total composition. Preferably the salt composition of the invention comprises glycerine in an amount of at least 5% by weight of the total composition. Preferably the salt composition of the invention comprises glycerine in an amount of at least 7% by weight of the total composition. Preferably the salt composition of the invention comprises glycerine in an amount of no greater than 30% by weight of the total composition. Preferably the salt composition of the invention comprises glycerine in an amount of no greater than 20% by weight of the total composition. Preferably the salt composition of the invention comprises glycerine in an amount of no greater than 15% by weight of the total composition. Preferably the salt composition of the invention comprises glycerine in an amount of no greater than 10% by weight of the total composition. Preferably the salt composition of the invention comprises glycerine in an amount of 1-30% by weight of the total composition. Preferably the salt composition of the invention comprises glycerine in an amount of 5-20% by weight of the total composition. Preferably the salt composition of the invention comprises glycerine in an amount of 7-15% by weight of the total composition. Preferably the salt composition of the invention comprises glycerine in an amount of 7-10% by weight of the total composition.

Preferably the salt composition of the invention comprises a surfactant, preferably an anionic surfactant. The surfactant may be present in any amount required to provide the desired cleaning effect. The surfactant is selected to act as detergent. Preferably the surfactant (preferably detergent) is present in an amount of at least 1% by weight of the total composition. Preferably the surfactant (preferably detergent) is present in an amount of at least 2% by weight of the total composition. Preferably the surfactant (preferably detergent) is present in an amount of at least 3% by weight of the total composition. Preferably the surfactant (preferably detergent) is present in an amount of at least 4% by weight of the total composition. Preferably the surfactant (preferably detergent) is present in an amount of at least 5% by weight of the total composition. Preferably the surfactant (preferably detergent) is present in an amount of at least 6% by weight of the total composition. Preferably the surfactant (preferably detergent) is present in an amount of at least 7% by weight of the total composition. Preferably the surfactant (preferably detergent) is present in an amount of at least 8% by weight of the total composition. Preferably the surfactant (preferably detergent) is present in an amount of at least 9% by weight of the total composition. Preferably the surfactant (preferably detergent) is present in an amount of at least 10% by weight of the total composition.

Preferably the surfactant (preferably detergent) is present in an amount of no greater than 20% by weight of the total composition. Preferably the surfactant (preferably detergent) is present in an amount of no greater than 19% by weight of the total composition. Preferably the surfactant (preferably detergent) is present in an amount of no greater than 18% by weight of the total composition. Preferably the surfactant (preferably detergent) is present in an amount of no greater than 17% by weight of the total composition. Preferably the surfactant (preferably detergent) is present in an amount of no greater than 16% by weight of the total composition. Preferably the surfactant (preferably detergent) is present in an amount of no greater than 15% by weight of the total composition. Preferably the surfactant (preferably detergent) is present in an amount of no greater than 14% by weight of the total composition. Preferably the surfactant (preferably detergent) is present in an amount of no greater than 13% by weight of the total composition. Preferably the surfactant (preferably detergent) is present in an amount of no greater than 12% by weight of the total composition. Preferably the surfactant (preferably detergent) is present in an amount of no greater than 11% by weight of the total composition. Preferably the surfactant (preferably detergent) is present in an amount of no greater than 10% by weight of the total composition.

Preferably the salt composition of the invention comprises a surfactant (preferably detergent). The surfactant (preferably detergent) may be present in any amount required to provide the desired emulsion. Preferably the surfactant (preferably detergent) is present in an amount of 1-20% by weight of the total composition. Preferably the surfactant (preferably detergent) is present in an amount of at least 2-19% by weight of the total composition. Preferably the surfactant (preferably detergent) is present in an amount of at least 3-18% by weight of the total composition. Preferably the surfactant (preferably detergent) is present in an amount of at least 4-16% by weight of the total composition. Preferably the surfactant (preferably detergent) is present in an amount of at least 5-15% by weight of the total composition. Preferably the surfactant (preferably detergent) is present in an amount of at least 6-14% by weight of the total composition. Preferably the surfactant (preferably detergent) is present in an amount of at least 7-13% by weight of the total composition. Preferably the surfactant (preferably detergent) is present in an amount of at least 7-12% by weight of the total composition. Preferably the surfactant (preferably detergent) is present in an amount of at least 7-11% by weight of the total composition. Preferably the surfactant (preferably detergent) is present in an amount of at least 8-11% by weight of the total composition. Preferably the surfactant (preferably detergent) is present in an amount of at least 8-10% by weight of the total composition. Preferably the surfactant (preferably detergent) is present in an amount of at least 9-10% by weight of the total composition.

The surfactant may be selected from suitable surfactants. Preferably the surfactant is selected from sodium lauryl sulphate, sodium laureth sulfate, ammonium lauryl sulphate, ammonium laureth sulfate, sodium cocoamphoacetate, disodium laureth sulfosuccinate, soap, sodium stearate, lauryl betaine and mixtures thereof. Preferably the surfactant is selected from sodium laureth sulfate, cocamide diethanolamine, lauryl betaine, and mixtures thereof. One preferred surfactant is sodium laureth sulfate.

The saturated fats the salt composition of the invention, if present, may be provided from a number of sources of cosmetically acceptable fats. In one aspect, the saturated fats are provided by cocoa butter, olive oil, mango butter, coconut oil, Japan wax, avocado butter and mixtures thereof. In one aspect, the saturated fats are provided by mango butter, avocado butter and mixtures thereof.

Each of the above preferred aspects of the present invention may be combined to provide a preferred salt composition of the invention. Each of the above preferred aspects of the present invention described herein with reference to 'scrub' may be applied to the salt composition of the invention to provide a preferred salt composition of the invention.

### Mask

In one preferred aspect, the cosmetic composition is a mask.

Preferably the cosmetic composition (such as a mask) comprises a colloidal dispersion of a clay. The clay may be selected from suitable clays. Preferably the clay is selected from kaolin, talc, calamine, rhassoul mud, fullers earth, bentonite clays and mixtures thereof.

Preferably the cosmetic composition (such as a mask) comprises clay in an amount of at least 5 % by weight of the total composition. Preferably the cosmetic composition (such as a mask) comprises clay in an amount of at least 10 % by weight of the total composition. Preferably the cosmetic composition (such as a mask) comprises clay in an amount of at least 15 % by weight of the total composition. Preferably the cosmetic composition (such as a mask) comprises clay in an amount of at least 20 % by weight of the total composition. Preferably the cosmetic composition (such as a mask) comprises clay in an amount of at least 25 % by weight of the total composition. Preferably the cosmetic composition (such as a mask) comprises clay in an amount of at least 30 % by weight of the total composition. Preferably the cosmetic composition (such as a mask) comprises clay in an amount of at least 33 % by weight of the total composition.

Preferably the cosmetic composition (such as a mask) comprises clay in an amount of no greater than 90 % by weight of the total composition. Preferably the cosmetic composition (such as a mask) comprises clay in an amount of no greater than 80 % by weight of the total composition. Preferably the cosmetic composition (such as a mask) comprises clay in an amount of no greater than 70 % by weight of the total composition. Preferably the cosmetic composition (such as a mask) comprises clay in an amount of no greater than 60 % by weight of the total composition. Preferably the cosmetic composition (such as a mask) comprises clay in an amount of no greater than 55 % by weight of the total composition. Preferably the cosmetic composition (such as a mask) comprises clay in an amount of no greater than 50 % by weight of the total composition. Preferably the cosmetic composition (such as a mask) comprises clay in an amount of no greater than 45 % by weight of the total composition. Preferably the cosmetic composition (such as a mask) comprises clay in an amount of no greater than 40 % by weight of the total composition. Preferably the cosmetic composition (such as a mask) comprises clay in an amount of no greater than 35 % by weight of the total composition.

Preferably the cosmetic composition (such as a mask) comprises clay in an amount of from 5 to 90% by weight of the total composition. Preferably the cosmetic composition (such as a mask) comprises clay in an amount of from 10 to 80% by weight of the total composition. Preferably the cosmetic composition (such as a mask) comprises clay in an amount of from 15 to 70% by weight of the total composition. Preferably the cosmetic composition (such as a mask) comprises clay in an amount of from 15 to 65% by weight of the total composition. Preferably the cosmetic composition (such as a mask) comprises clay in an amount of from 15 to 60% by weight of the total composition. Preferably the cosmetic composition (such as a mask) comprises clay in an amount of from 15 to 55% by weight of the total composition. Preferably the cosmetic composition (such as a mask) comprises clay in an amount of from 15 to 50% by weight of the total composition. Preferably the cosmetic composition (such as a mask) comprises clay in an amount of from 25 to 40% by weight of the total composition. Preferably the cosmetic composition (such as a mask) comprises clay in an amount of from 30 to 35% by weight of the total composition. Preferably the cosmetic composition (such as a mask) comprises water in an amount of at least 5 % by weight of the total composition. Preferably the cosmetic composition (such as a mask) comprises water in an amount of at least 8 % by weight of the total composition. Preferably the cosmetic composition (such as a mask) comprises water in an amount of at least 10 % by weight of the total composition. Preferably the cosmetic composition (such as a mask) comprises water in an amount of at least 11 % by weight of the total composition. Preferably the cosmetic composition (such as a mask) comprises water in an amount of at least 12 % by weight of the total composition. Preferably the cosmetic composition (such as a mask) comprises water in an amount of at least 13 % by weight of the total composition.

Preferably the cosmetic composition (such as a mask) comprises water in an amount of no greater than 30 % by weight of the total composition. Preferably the cosmetic composition (such as a mask) comprises water in an amount of no greater than 25 % by weight of the total composition. Preferably the cosmetic composition (such as a mask) comprises water in an amount of no greater than 20 % by weight of the total composition. Preferably the cosmetic composition (such as a mask) comprises water in an amount of no greater than 18 % by weight of the total composition. Preferably the cosmetic composition (such as a mask) comprises water in an amount of no greater than 15 % by weight of the total composition. Preferably the cosmetic composition (such as a mask) comprises water in an amount of no greater than 13 % by weight of the total composition.

Preferably the cosmetic composition (such as a mask) comprises water in an amount of 5 to 30 % by weight of the total composition. Preferably the cosmetic composition (such as a mask) comprises water in an amount of 10 to 30 % by weight of the total composition. Preferably the cosmetic composition (such as a mask) comprises water in an amount of from 10 to 25% by weight of the total composition, such as in an amount of from 10 to 20% by weight of the total composition, such as in an amount of from 12 to 18% by weight of the total composition.

Preferably the cosmetic composition (such as a mask) comprises a humectant. The humectant may be selected from suitable humectants. Preferably the humectant is selected from honey, glycerine, mono propylene glycol (1,2-propandiol), agave nectar, fruit syrups, herbal syrups, sugar solutions and mixtures thereof. Preferably the humectant is selected from honey, glycerine and mixtures thereof.

Preferably the cosmetic composition (such as a mask) comprises humectant in an amount of at least 5 % by weight of the total composition. Preferably the cosmetic composition (such as a mask) comprises humectant in an amount of at least 10 % by weight of the total composition. Preferably the cosmetic composition (such as a mask) comprises humectant in an amount of at least 15 % by weight of the total composition. Preferably the cosmetic composition (such as a mask) comprises humectant in an amount of at least 20 % by weight of the total composition. Preferably the cosmetic composition (such as a mask) comprises humectant in an amount of at least 25 % by weight of the total composition. Preferably the cosmetic composition (such as a mask) comprises humectant in an amount of at least 30 % by weight of the total composition. Preferably the cosmetic composition (such as a mask) comprises humectant in an amount of at least 35 % by weight of the total composition. Preferably the cosmetic composition (such as a mask) comprises humectant in an amount of at least 40 % by weight of the total composition. Preferably the cosmetic composition (such as a mask) comprises humectant in an amount of at least 42 % by weight of the total composition.

Preferably the cosmetic composition (such as a mask) comprises humectant in an amount of no greater than 90 % by weight of the total composition. Preferably the cosmetic composition (such as a mask) comprises humectant in an amount of no greater than 80 % by weight of the total composition. Preferably the cosmetic composition (such as a mask) comprises humectant in an amount of no greater than 70 % by weight of the total composition. Preferably the cosmetic composition (such as a mask) comprises humectant in an amount of no greater than 60 % by weight of the total composition. Preferably the cosmetic composition (such as a mask) comprises humectant in an amount of no greater than 55 % by weight of the total composition. Preferably the cosmetic composition (such as a mask) comprises humectant in an amount of no greater than 50% by weight of the total composition. Preferably the cosmetic composition (such as a mask) comprises humectant in an amount of no greater than 45% by weight of the total composition. Preferably the cosmetic composition (such as a mask) comprises humectant in an amount of no greater than 43 % by weight of the total composition.

Preferably the cosmetic composition (such as a mask) comprises a humectant in an amount of 25 to 60 % by weight of the total composition. Preferably the cosmetic composition (such as a mask) comprises humectant in an amount of 30% to 50% by weight of the total composition, such as in an amount of 37% to 47% by weight of the total composition.

Preferably the cosmetic composition (such as a mask) comprises
(a) a colloidal dispersion of a clay;
(b) water in an amount of 10 to 30 % by weight of the total composition; and
(c) humectant in an amount of 25 to 60 % by weight of the total composition.

Preferably the cosmetic composition (such as a mask) comprises
(a) a colloidal dispersion of a clay wherein the clay is present in an amount of from 25 to 40% by weight of the total composition;
(b) water in an amount of 12 to 18 % by weight of the total composition; and
(c) humectant in an amount of 37 to 47 % by weight of the total composition.

We have found that by preparing a formulation within certain parameters a cosmetic composition may be prepared which does not require the presence of preservatives (as described herein). Thus in a further broad aspect the present invention provides a cosmetic composition comprising
(a) a colloidal dispersion of a clay;
(b) water in an amount of 10 to 30 % by weight of the total composition; and
(c) humectant in an amount of 25 to 60 % by weight of the total composition.

This system is referred to as the 'clay composition of the invention'. It is understood that by producing such a formulation a cosmetic composition is formed which inhibits the growth of microorganisms. The specific combination of clay and humectant acts to maintain the level of active water so low as to inhibit the growth of microorganisms. Furthermore the presence of the clay results in the dispersion of water within the colloidal matrix. This dispersion of the water across the matrix further inhibits the growth of microorganisms. Thus a system is provided which is considered to be "self preserving" - growth of microorganisms is inhibited without the use of an additional preservative. Therefore, the clay composition may be free from preservatives, wherein free from preservatives is as defined herein.

The clay may be selected from suitable clays. Preferably the clay is selected from kaolin, talc, calamine, rhassoul mud, fullers earth, bentonite clays and mixtures thereof.

Preferably the clay composition comprises clay in an amount of at least 5 % by weight of the total composition. Preferably the clay composition comprises clay in an amount of at least 10 % by weight of the total composition. Preferably the clay composition comprises clay in an amount of at least 15 % by weight of the total composition. Preferably the clay composition comprises clay in an amount of at least 20 % by weight of the total composition. Preferably the clay composition comprises clay in an amount of at least 25 % by weight of the total composition. Preferably the clay composition comprises clay in an amount of at least 30 % by weight of the total composition. Preferably the clay composition comprises clay in an amount of at least 33 % by weight of the total composition.

Preferably the clay composition comprises clay in an amount of no greater than 90 % by weight of the total composition. Preferably the clay composition comprises clay in an amount of no greater than 80 % by weight of the total composition. Preferably the clay composition comprises clay in an amount of no greater than 70 % by weight of the total composition. Preferably the clay composition comprises clay in an amount of no greater than 60 % by weight of the total composition. Preferably the clay composition comprises clay in an amount of no greater than 55 % by weight of the total composition. Preferably the clay composition comprises clay in an amount of no greater than 50 % by weight of the total composition. Preferably the clay composition comprises clay in an amount of no greater than 45 % by weight of the total composition. Preferably the clay composition comprises clay in an amount of no greater than 40 % by weight of the total composition. Preferably the clay composition comprises clay in an amount of no greater than 35 % by weight of the total composition.

Preferably the clay composition comprises clay in an amount of from 5 to 90% by weight of the total composition. Preferably the clay composition comprises clay in an amount of from 10 to 80% by weight of the total composition. Preferably the clay composition comprises clay in an amount of from 15 to 70% by weight of the total composition. Preferably the clay composition comprises clay in an amount of from 15 to 65% by weight of the total composition. Preferably the clay composition comprises clay in an amount of from 15 to 60% by weight of the total composition. Preferably the clay composition comprises clay in an amount of from 15 to 55% by weight of the total composition. Preferably the clay composition comprises clay in an amount of from 15 to 50% by weight of the total composition. Preferably the clay composition comprises clay in an amount of from 25 to 40% by weight of the total composition. Preferably the clay composition comprises clay in an amount of from 30 to 35% by weight of the total composition. Preferably the clay composition comprises water in an amount of at least 5 % by weight of the total composition. Preferably the clay composition comprises water in an amount of at least 8 % by weight of the total composition. Preferably the clay composition comprises water in an amount of at least 10 % by weight of the total composition. Preferably the clay composition comprises water in an amount of at least 11 % by weight of the total composition. Preferably the clay composition comprises water in an amount of at least 12 % by weight of the total composition. Preferably the clay composition comprises water in an amount of at least 13 % by weight of the total composition.

Preferably the clay composition comprises water in an amount of no greater than 30 % by weight of the total composition. Preferably the clay composition comprises water in an amount of no greater than 25 % by weight of the total composition. Preferably the clay composition comprises water in an amount of no greater than 20 % by weight of the total composition. Preferably the clay composition comprises water in an amount of no greater than 18 % by weight of the total composition. Preferably the clay composition comprises water in an amount of no greater than 15 % by weight of the total composition. Preferably the clay composition comprises water in an amount of no greater than 13 % by weight of the total composition.

Preferably the clay composition comprises water in an amount of 5 to 30 % by weight of the total composition. Preferably the clay composition comprises water in an amount of 10 to 30 % by weight of the total composition. Preferably the clay composition comprises water in an amount of from 10 to 25% by weight of the total composition, such as in an amount of from 10 to 20% by weight of the total composition, such as in an amount of from 12 to 18% by weight of the total composition.

Preferably the clay composition comprises a humectant. The humectant may be selected from suitable humectants. Preferably the humectant is selected from honey, glycerine, mono propylene glycol (1,2-propandiol), agave nectar, fruit syrups, herbal syrups, sugar solutions and mixtures thereof. Preferably the humectant is selected from honey, glycerine and mixtures thereof.

Preferably the clay composition comprises humectant in an amount of at least 5 % by weight of the total composition. Preferably the clay composition comprises humectant in an amount of at least 10 % by weight of the total composition. Preferably the clay composition comprises humectant in an amount of at least 15 % by weight of the total composition. Preferably the clay composition comprises humectant in an amount of at least 20 % by weight of the total composition. Preferably the clay composition comprises humectant in an amount of at least 25 % by weight of the total composition. Preferably the clay composition comprises humectant in an amount of at least 30 % by weight of the total composition. Preferably the clay composition comprises humectant in an amount of at least 35 % by weight of the total composition. Preferably the clay composition comprises humectant in an amount of at least 40 % by weight of the total composition. Preferably the clay composition comprises humectant in an amount of at least 42 % by weight of the total composition.

Preferably the clay composition comprises humectant in an amount of no greater than 90 % by weight of the total composition. Preferably the clay composition comprises humectant in an amount of no greater than 80 % by weight of the total composition. Preferably the clay composition comprises humectant in an amount of no greater than 70 % by weight of the total composition. Preferably the clay composition comprises humectant in an amount of no greater than 60 % by weight of the total composition. Preferably the clay composition comprises humectant in an amount of no greater than 55 % by weight of the total composition. Preferably the clay composition comprises humectant in an amount of no greater than 50% by weight of the total composition. Preferably the clay composition comprises humectant in an amount of no greater than 45% by weight of the total composition. Preferably the clay composition comprises humectant in an amount of no greater than 43 % by weight of the total composition.

Preferably the clay composition comprises a humectant in an amount of 25 to 60 % by weight of the total composition. Preferably the clay composition comprises humectant in an amount of 30% to 50% by weight of the total composition, such as in an amount of 37% to 47% by weight of the total composition.

Preferably the clay composition comprises
(a) a colloidal dispersion of a clay wherein the clay is present in an amount of from 25 to 40% by weight of the total composition;
(b) water in an amount of 12 to 18 % by weight of the total composition; and
(c) humectant in an amount of 37 to 47 % by weight of the total composition.

### Further Components

Fragrance may be added to the product to make the experience of using the present composition more pleasant. Combining essential oils such as lavender, chamomile or rose absolute into fragrances for the invention ensures the user has a pleasant washing experience.

The amount of fragrances is preferably from about 0.1% to about 10% by weight of the total composition, such as from about 0.1% to about 5% by weight of the total composition, such as from about 0.1% to about 4% by weight of the total composition, such as from about 0.5% to about 5% by weight of the total composition, such as from about 1% to about 5% by weight of the total composition, such as from about 0.5% to about 4% by weight of the total composition, such as from about 0.5% to about 3% by weight of the total composition, such as from about 0.5% to about 2% by weight of the total composition, such as from about 0.5% to about 1.5% by weight of the total composition.

The essential oils may be selected based on the fragrance desired, skin type to be treated and other effects desired based on the well-known properties of essential oils. The addition of essential oils, when taken in to the nose, are known to alter mood. For example, essential oils are known to create effects of drowsiness or stimulating the senses. Many well documented effects can be achieved by the use of essential oils.

In one embodiment, the one or more essential oils present in the product are selected from Tarragon, Lemon myrtle, Jasmin, Ylang ylang, Labdanum, Lemongrass, Rose otto, Grapefruit, Patchouli, Rosemary, Armois, Lemon, Neroli, Sweet violet, Lavender, Orange 50 fold, Vanilla, Peppermint, Benzoin, Hydrangia, Litsea Cubeba, Cardamon, Tonka, and Chamomile blue. In one embodiment, the one or more essential oils present in the product are selected from Tarragon, Lemon myrtle, Labdunum, and Lemon.

Vitamins, particularly B, C and E are very beneficial for the skin. Vitamin rich ingredients such as Wheatgerm oil can also be used to deliver vitamins on to the skin. In a one embodiment, the vitamins are selected from vitamin B, vitamin C, vitamin E and mixtures thereof. It will be appreciated by one skilled in the art that the vitamin may be provided from any suitable source. For example the vitamin(s) may be provided from a synthetic source or from incorporation into the product of a material, such as a natural material, that has a high vitamin content.

The advantages of the present invention discussed herein may be applied to any cosmetic product, for example to any emulsion or dispersion. Other cosmetic categories that would be applicable under the present invention include:
shaving preparations, shower gels & shower jellies, moisturisers, skincare & body lotions, sunscreen products, shampoos, conditioners & hair dressings, face masks and lip balms. All are categories of product, which conventionally require packaging, are stored at room temperature and have a long shelf life.

Materials which may be included in the present composition include but are not limited to:
Water Phase including - Water, Plant Infusions & Decoctions (including Tea & Coffee), Fruit & Vegetable Juices, Whole Fruit & Vegetables, Vinegar, Beers, Wines & Spirits - typically used in a range of 0.1% - 70% for leave-on products and 5% - 65% for wash-off products.
Humectants including Honey, Glycerine, Mono Propylene Glycol, 1,2 Propandiol, Agave Nectar, Fruit Syrups, Herbal Syrups, Sugar solutions - typically used in a range of 0.1%-45%
Emulsifiers including - emulsifying solvents such as Triethanolamine & Lactic Acid, emulsifying waxes such as Stearic Acid, Cetearyl Alcohol, Glyceryl Stearate, Cetyl Alcohol / Sodium Laureth Sulfate, Glyceryl Stearate & Polyethylene Glycol 100 - typically used in a range of 1.5% - 25%
Surfactants (for wash-off products only) including but not limited too - Sodium Lauryl / Laureth Sulfate, Ammonium Lauryl / Laureth Sulfate, Sodium Cocoamphoacetate, Disodium Laureth Sulfosuccinate, Soap, Sodium Stearate, Lauryl Betaine - typically used in a range of 4% - 35%
Oil, Butter & Waxes - Almond Oil, Sesame Oil, Evening Primrose Oil, Jojoba Oil, Cocoa Butter, Shea Butter, Mango Butter, Cupuacu Butter, Lanolin, Beeswax, Rose Wax, Orange Peel Wax - typically used in a range of 5% - 70%.
Whole Fruits & Vegetables - Avocado, Banana, Strawberries, Blueberries, typically used in a range of 0.5% - 25%.
Fragrance Materials - typically used in a range of 0.1% - 5%.
Colorant Materials - typically used in a range of 0.001 % - 2%
Sunscreens - Octocrylene, Titanium Dioxide, Ethylhexyl Methoxycinnamate - typically used in a range of 3% - 25%.
Clays - Kaolin, Talc, Calamine, Rhassoul Mud, Fullers Earth, Bentonite Clays and preparations thereof. - typically used in a range of 0.1% - 40%
Salts & Sugars - Sea Salt, Castor Sugar, Granulated Sugar, Brown Sugar, Molasses - typically used in a range of 0.1% - 50%
Herbs, Cereals and Beans - Oats, Rice, Cinnamon, Vanilla, Aduki Beans, Seaweeds - typically used in a range of 0.01% - 15%
Gelling & Film Forming Agents - Agar Agar, Carrageenan, PVP - typically used in a range of 0.5% - 12%
Stabilising Agents - Lactic Acid, Ascorbic Acid, Malic Acid & Fruit Powder - typically used in a range of 0.01% - 3%.
Protein Sources - Tofu, Banana, Soya, Soya Lecithin, Eggs - typically used in a range of 1% - 30%.

### Examples

The invention will now be described with reference to the following non-limiting examples.

### Example 1a - Emulsion Lotion

A product of total batch size of 1,000g having the following composition was prepared.

| **Phase** | **Formula % (wt.)** | **Raw Material Type** |
|---|---|---|
| | | |
| **A** | 33.000 | Water-Tap |
| | 1.00 | Fine Ground Almonds |
| | | |
| **B** | 10.00 | Glycerine-PF |
| | 1.000 | Triethanolamine 99% |
| | | |
| **C** | 40.70 | Almond Oil |
| | 3.30 | Stearic Acid |
| | 1.00 | Cetearyl Alcohol |
| | 9.50 | Cocoa Butter |
| **D** | 0.50 | Fragrance. |
| | | |
| | 100.00 | |

### Method

1. Heat the Water to boiling point. Add the Almonds. Filter and reweigh the content to the former percentage of water.
2. Add the ingredients in phase B to phase A and warm to 68 centigrade.
3. Warm the oil based ingredients in C to 68 centigrade.
4. Add phases A. B to phase C, emulsify and cool.
5 Add the Fragrance.

The product is found to be microbiologically stable during storage.

### Example 1b - Emulsion Lotion

A product of total batch size of 1,000g having the following composition was prepared.

| **Phase** | **Formula % (wt.)** | **Raw Material Type** |
|---|---|---|
| | | |
| **A** | 50.000 | Water-Tap |
| | 7.00 | Glycerine-PF |
| | 1.000 | Triethanolamine 99% |
| | | |
| **B** | 29.80 | Almond Oil |
| | 2.50 | Stearic Acid |
| | 0.70 | Cetearyl Alcohol |
| | 9.00 | Cocoa Butter |

### Method

1. Mix Phase A components together and heat to 75°C.
2. Mix Phase B components and heat to 75°C.
3. Combine the ingredients in phase B and phase A and emulsify
4. Cool.

The product is found to be microbiologically stable during storage.

### Example 1c - Emulsion Lotion

A product of total batch size of 1,000g having the following composition was prepared.

| **Phase** | **Formula % (wt.)** | **Raw Material Type** |
|---|---|---|
| | | |
| **A** | 60.000 | Water-Tap |
| | 7.00 | Glycerine-PF |
| | 1.000 | Triethanolamine 99% |
| | | |
| **B** | 20.00 | Almond Oil |
| | 2.50 | Stearic Acid |
| | 0.50 | Cetearyl Alcohol |
| | 9.00 | Cocoa Butter |

### Method

1. Mix Phase A components together and heat to 75°C.
2. Mix Phase B components and heat to 75°C.
3. Combine the ingredients in phase B and phase A and emulsify
4. Cool.

The product is found to be microbiologically stable during storage.

### Example 1d - Emulsion Lotion

A product of total batch size of 1,000g having the following composition was prepared.

| **Phase** | **Formula % (wt.)** | **Raw Material Type** |
|---|---|---|
| | | |
| **A** | 70.000 | Water-Tap |
| | 7.00 | Glycerine-PF |
| | 1.000 | Triethanolamine 99% |
| | | |
| **B** | 10.00 | Almond Oil |
| | 2.50 | Stearic Acid |
| | 0.50 | Cetearyl Alcohol |
| 9.00 | | Cocoa Butter |

### Method

1. Mix Phase A components together and heat to 75°C.
2. Mix Phase B components and heat to 75°C.
3. Combine the ingredients in phase B and phase A and emulsify
4. Cool.

The product is found to be microbiologically stable during storage.

### Example 2 - Mask

A product of total batch size of 1,000g having the following composition was prepared.

| **Phase** | **Formula % (wt.)** | **Raw Material Type** |
|---|---|---|
| | | |
| **A** | 13.60 | Water |
| | 0.30 | Bentonite Clay |
| | | |
| **B** | 21.40 | Glycerine-BP-Vegetable Grade |
| | 21.40 | Honey-Clear |
| | 0.30 | Fragrance |
| | | |
| **C** | 12.00 | Kaolin |
| | 21.00 | Calamine |
| | | |
| **D** | 10.00 | Fine Ground Almonds |
| | | |
| | 100.00 | |

### Method

1. Add the Bentonite Clay to the water & emulsify.
2. Add the ingredients in phase B in the order listed.
3. Add phase C in the order listed. Stir in gently.
4. Add phase D & mix to a smooth consistency.

The product is found to be microbiologically stable during storage.

### Example 3 - Face Wash/Facial Scrub

A product of total batch size of 1,000g having the following composition was prepared.

| | **Formula % (wt.)** | **Raw Material Type** |
|---|---|---|
| **A** | 6.00 | Whole Fresh Lemon |
| | 7.00 | Water |
| | 7.56 | Glycerine |
| | | |
| **B** | 1.19 | Triethanolamine |
| | | |
| **C** | 2.75 | Avocado Butter-Organic (saturated fat) |
| | 2.75 | Mango Butter (saturated fat) |
| | 1.50 | Jojoba oil (unsaturated oil) |
| | 6.00 | Stearic acid |
| | 3.50 | Cetearyl Alcohol |
| | 10.00 | Sodium Laureth Sulphate |
| | | |
| **D** | 1.00 | Fragrance |
| | | |
| **E** | | ***Colour Solution*** |
| | 0.112500 | Water-Tap |
| | 0.0125 | Colourant |
| | 0.625 | Glycerine |
| | | |
| **F** | 50.00 | Salt-Fine |
| | 100.00 | |

### Method

1. Liquidise the lemon with the water content and glycerine.
2. Add this to phase B and warm to 68 degrees centigrade.
3. Warm C to 68 degrees centigrade.
4. Add A.B to C. and emulsify gently.
5. Cool and add D. Prepare E and add.
6. Gradually add the salt.

The product is found to be microbiologically stable during storage.

### Example 4 - Microbiological Testing of Emulsion/Lotion

All microbiological testing was conducted by a contract UKAS accredited microbiology laboratory.

Two groups of samples were taken from the emulsion formulation described in Example 1a, immediately after its manufacture. One group of samples of the product were tested by volunteer for a period of two weeks, where the volunteers applied the lotion to their skin daily. The test volunteers returned the used product after two weeks of use. The used sample was sent for microbiological testing, under controlled conditions, after a total period of 6 weeks after manufacture.

The second group of samples of the product was stored under ambient conditions for a period of two years, available for re-testing if needed. At the end of the two year period, the second group of samples was discarded.

Multiple tests were run on samples of the product, both used samples and stored samples.

| Total no. of Tests | Aerobic Plate Count - 30°C/72 hrs (cfu/g) | Yeasts (cfu/g) | Moulds (cfu/g) |
|---|---|---|---|
| 24 | <10-40 (*Bacillus* ssp.)* | <10 | ≤10 |

| | | | |
|---|---|---|---|
| * Confirmed identification of organisms is outside the scope of the laboratory UKAS accreditation. | | | |

Therefore, it was found that the Emulsion Lotion of Example 1a is microbiologically stable during storage and after use.

### Example 5 - Microbiological Testing of Mask

All microbiological testing was conducted by a contract UKAS accredited microbiology laboratory, as described for Example 4.

Three samples were taken from the mask formulation described in Example 2, immediately after its manufacture. One sample of the product was sent for microbiological testing, under controlled conditions.

The second sample of the product was stored under refrigerated conditions (5-8°C) for a total period of three months, available for re-testing if needed. At the end of the three month period, the second sample was discarded.

A number of these tests were run on different samples of the product.

| Total no. of Tests | Aerobic Plate Count - 30°C/72 hrs (cfu/g) | Yeasts (cfu/g) | Moulds (cfu/g) |
|---|---|---|---|
| 9 | 50 - 1.8x10² (*Bacillus* ssp.)* | <10 | <10 |

| | | | |
|---|---|---|---|
| * Confirmed identification of organisms is outside the scope of the laboratory UKAS accreditation. | | | |

The third sample of the product was tested by a number of test volunteers for a period of two weeks, where the volunteers applied the mask to their skin daily. The test volunteers returned the used product after two weeks of use. The used products were sent for microbiological testing, under controlled conditions, after a total period of 7-10 weeks after manufacture.

| Total no. of Tests | Aerobic Plate Count - 30°C/72 hrs (cfu/g) | Yeasts (cfu/g) | Moulds (cfu/g) |
|---|---|---|---|
| 6 | 40 - 2.6x10^{2 a} (*Bacillus* ssp.)* | ≤10 | <10-20 ^{b} |

| | | | |
|---|---|---|---|
| * Confirmed identification of organisms is outside the scope of the laboratory UKAS accreditation. ^{a} One sample that was tested after a total period of 7 weeks storage was an outlier with an Aerobic Plate Count of 9.8x10³ cfu/g. ^{b} One sample that was tested after a total period of 10 weeks was an outlier with the amount of mould present in the sample being 1.4x10² cfu/g; this is clearly an anomaly based on the results of all other test samples. | | | |

Therefore, it has been demonstrated that the Mask formulation of Example 2 is microbiologically stable, both immediately after manufacture, and after storage and use.

### Example 6 - Microbiological Testing of Face Wash/Facial Scrub

All microbiological testing was conducted by a contract UKAS accredited microbiology laboratory, as described for Example 4.

Three samples were taken from the scrub formulation described in Example 3, immediately after its manufacture. One sample of the product was sent for microbiological testing, under controlled conditions.

The second sample of the product was stored under ambient conditions (5-8°C) for a total period of two years, available for re-testing if needed. At the end of the two year period, the second sample was discarded.

A number of the microbiological tests were run on different samples of the product, after a total period of 19 weeks after manufacture.

| Total no. of Tests | Aerobic Plate Count - 30°C/72 hrs (cfu/g) | Yeasts (cfu/g) | Moulds (cfu/g) |
|---|---|---|---|
| 53 | <10 - 70^{c} (*Bacillus* ssp.)** | <10 | <10-20 ^{d} |

| | | | |
|---|---|---|---|
| * Confirmed identification of organisms is outside the scope of the laboratory UKAS accreditation. ^{c} One sample was an outlier with an Aerobic Plate Count of 1.8x10² cfu/g; this is thought to be due to the presence of Micrococci in the sample in addition to the *Bacillus* ssp. detected in some of the other samples. Micrococci were not detected in any of the other 52 samples tested, and therefore this result was clearly an anomaly, and the aerobic plate count can be considered to have been in the range of from <10 to 70 cfu/g. ^{d} As above, one sample was an outlier with the amount of mould present in the sample being 90 cfu/g. | | | |

The third sample of the product was tested by a number of test volunteers for a period of two weeks, where the volunteers applied the scrub to their skin daily. The test volunteers returned the used product after two weeks of use. The used products were sent for microbiological testing, under controlled conditions, after a total period of 7-10 weeks after manufacture.

| Total no. of Tests | Aerobic Plate Count - 30°C/72 hrs (cfu/g) | Yeasts (cfu/g) | Moulds (cfu/g) |
|---|---|---|---|
| 3 | <10 | <10 | <10 |

Therefore, it has been demonstrated that the Face Wash/Facial Scrub formulation of Example 3 is microbiologically stable, both after storage and after daily use for two weeks.

The invention will now be described in further detail in the following numbered paragraphs:
1. A cosmetic composition comprising
   (i) water in an amount of from 5 to 70% by weight of the total composition;
      wherein the cosmetic composition is free from preservatives, wherein free from preservatives is as defined herein.
2. A cosmetic composition according to paragraph 1, wherein water is present in an amount of from 5 to 50% by weight of the total composition;
3. A cosmetic composition according to paragraph 1 or 2, wherein water is present in an amount of from 10 to 50% by weight of the total composition.
4. A cosmetic composition according to any one of the preceding paragraphs wherein water is present in an amount of from 25 to 45% by weight of the total composition.
5. A cosmetic composition according to any one of the preceding paragraphs wherein water is present in an amount of from 30 to 40% by weight of the total composition.
6. A cosmetic composition according to any one of the preceding paragraphs wherein water is present in an amount of from 32 to 38% by weight of the total composition.
7. A cosmetic composition according to any one of the preceding paragraphs further comprising saturated fats in an amount of 9% to 15% by weight of the total composition.
8. A cosmetic composition according to paragraph 1 comprising
   (i) water in an amount of from 20 to 70% by weight of the total composition;
   (ii) saturated fats in an amount of 9% to 15% by weight of the total composition.
9. A cosmetic composition according to paragraph 6, wherein saturated fats are present in an amount of 9% to 11% by weight of the total composition.
10. A cosmetic composition according to paragraph 7, 8 or 9, wherein the saturated fats are provided by cocoa butter, olive oil and mixtures thereof.
11. A cosmetic composition according to any one of paragraphs 7 to 10, comprising cocoa butter in an amount of at least 6% by weight of the total composition
12. A cosmetic composition according to any one of paragraphs 7 to 11, comprising cocoa butter in an amount of no greater than 15% by weight of the total composition
13. A cosmetic composition according to any one of paragraphs 7 to 12, comprising olive oil in an amount of at least 25% by weight of the total composition
14. A cosmetic composition according to any one of paragraphs 7 to 13, comprising olive oil in an amount of no greater than 50% by weight of the total composition
15. A cosmetic composition according to paragraph 1, wherein water is present in an amount of from 10 to 20% by weight of the total composition.
16. A cosmetic composition according to paragraph 15, wherein water is present in an amount of from 12 to 20% by weight of the total composition.
17. A cosmetic composition according to paragraph 15, wherein water is present in an amount of from 14 to 18% by weight of the total composition.
18. A cosmetic composition according to any one of the preceding paragraphs further comprising salt in an amount of 30% to 70% by weight of the total composition.
19. A cosmetic composition according to paragraph 18, wherein salt is present in an amount of 40% to 60% by weight of the total composition.
20. A cosmetic composition according to paragraph 18, wherein salt is present in an amount of 45% to 55% by weight of the total composition.
21. A cosmetic composition according to paragraph 1 comprising
   (i) water in an amount of from 5 to 30% by weight of the total composition;
   (ii) saturated fats in an amount of 2% to 15% by weight of the total composition; and
   (iii) salt in an amount of 30% to 90% by weight of the total composition.
22. A cosmetic composition according to paragraph 1 comprising
   (i) water in an amount of from 14 to 18% by weight of the total composition;
   (ii) saturated fats in an amount of 6.5% to 7.5% by weight of the total composition; and
   (iii) salt in an amount of 45% to 55% by weight of the total composition.
23. A cosmetic composition according to paragraph 7, 8 or 9, wherein the saturated fats are provided by mango butter, coconut oil, Japan wax, avocado butter and mixtures thereof.
24. A cosmetic composition according to any one of the preceding paragraphs, further comprising unsaturated fats in an amount of 2% to 10% by weight of the total composition.
25. A cosmetic composition according to paragraph 22 comprising unsaturated fats in an amount of 3% to 7% by weight of the total composition.
26. A cosmetic composition according to paragraph 1, comprising
   (a) a colloidal dispersion of a clay;
   (b) water in an amount of 10 to 30 % by weight of the total composition; and
   (c) humectant in an amount of 25 to 60 % by weight of the total composition.
27. A cosmetic composition according to paragraph 26, wherein the clay is present in an amount of from 15 to 55% by weight of the total composition.
28. A cosmetic composition according to paragraph 26 or 27, wherein the clay is present in an amount of from 25 to 40% by weight of the total composition.
29. A cosmetic composition according to paragraph 24, 25 or 26, wherein the clay is selected from kaolin, talc and mixtures thereof.
30. A cosmetic composition according to paragraph 1, wherein water is present in an amount of from 10 to 25% by weight of the total composition.
31. A cosmetic composition according to paragraph 30, wherein water is present in an amount of from 10 to 20% by weight of the total composition.
32. A cosmetic composition according to paragraph 30, wherein water is present in an amount of from 12 to 18% by weight of the total composition.
33. A cosmetic composition according to any one of paragraphs 1 to 25, further comprising a humectant.
34. A cosmetic composition according to paragraph 33 wherein the humectant is selected from honey, glycerine, monopropylene glycol, 1,2-propanediol, agave nectar, fruit syrups, herbal syrups, sugar solutions and mixtures thereof.
35. A cosmetic composition according to paragraph 34, wherein the humectant is selected from honey, glycerine and mixtures thereof.
36. A cosmetic composition according to paragraph 33, 34 or 35, wherein the humectant is present in an amount of from 0.1% to 50% by weight of the total composition.
37. A cosmetic composition according to any one of paragraphs 33 to 36, wherein humectant is present in an amount of 30% to 50% by weight of the total composition.
38. A cosmetic composition according to any one of paragraphs 33 to 37, wherein humectant is present in an amount of 37% to 47% by weight of the total composition.
39. A cosmetic composition according to paragraph 1 comprising
   (a) a colloidal dispersion of a clay wherein the clay is present in an amount of from 25 to 40% by weight of the total composition;
   (b) water in an amount of 12 to 18 % by weight of the total composition; and
   (c) humectant in an amount of 37 to 47 % by weight of the total composition.
40. A cosmetic composition according to any one of the preceding paragraphs, further comprising at least one additional component selected from binders, fillers, opacifiers, perfumes, colours, fragrances, scrubs, exfoliants and mixtures thereof.
41. A cosmetic composition according to paragraph 40, further comprising at least one additional component selected from colours, fragrances, scrubs, exfoliants and mixtures thereof.
42. A cosmetic composition according to any one of the preceding paragraphs, further comprising fruit selected from lime, grapefruit and mixtures thereof.
43. A cosmetic composition according to any one of the preceding paragraphs, further comprising an emulsifier.
44. A cosmetic composition according to paragraph 43, wherein the emulsifier is selected from triethanolamine, lactic acid, stearic acid, cetearyl alcohol, glyceryl stearate, cetyl alcohol, sodium laureth sulfate, glyceryl stearate, polyethylene glycol and mixtures thereof
45. A cosmetic composition according to paragraph 43, wherein the emulsifier is selected from stearic acid, cetearyl alcohol and mixtures thereof.
46. A cosmetic composition according to paragraph 43, 44 or 45, wherein the emulsifier is present in an amount of from 1.5% to 15% by weight of the total composition.
47. A cosmetic composition according to any one of the preceding paragraphs, further comprising a protein.
48. A cosmetic composition according to paragraph 47 wherein the protein is selected tofu, banana, soya, soya lecithin, eggs and mixtures thereof.
49. A cosmetic composition according to paragraph 47 or 48 wherein the protein is present in an amount of from 1% to 30% by weight of the total composition.
50. A cosmetic emulsion composition comprising
   (i) water in an amount of from 20 to 70% by weight of the total composition;
   (ii) saturated fats in an amount of 9% to 15% by weight of the total composition.
51. A cosmetic composition comprising
   (i) water in an amount of from 5 to 30% by weight of the total composition;
   (ii) saturated fats in an amount of 2% to 15% by weight of the total composition; and
   (iii) salt in an amount of 30% to 90% by weight of the total composition.
52. A cosmetic composition comprising
   (a) a colloidal dispersion of a clay;
   (b) water in an amount of 10 to 30 % by weight of the total composition; and
   (c) humectant in an amount of 25 to 60 % by weight of the total composition.
53. A process for the production of a cosmetic composition as defined in paragraphs 1 to 52 comprising the step of mixing:
   (i) water in an amount of from 5 to 70% by weight of the total composition; and
   (ii) cosmetically acceptable ingredients
      wherein the cosmetic composition is free from preservatives, wherein free from preservatives is as defined in the description.
54. A process according to paragraph 53 wherein the process excludes a step of addition one or more prohibited preservatives, wherein the prohibited preservatives are as defined in the description.
55. A product obtained or obtainable by the process of paragraph 54.
56. A cosmetic method comprising contacting the skin or hair of a user with a cosmetic composition as defined in paragraphs 1 to 52.
57. A cosmetic composition as substantially defined herein with reference to the examples.
58. A process as substantially defined herein with reference to the examples.
59. A cosmetic method as substantially defined herein with reference to the examples.

Various modifications and variations of the present invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry, biology or related fields are intended to be within the scope of the following claims.

## Claims

1. A cosmetic composition comprising
(i) water in an amount of from 5 to 30% by weight of the total composition;
(ii) saturated fats in an amount of 2% to 15% by weight of the total composition;
(iii) salt in an amount of 30% to 90% by weight of the total composition; and
(iv) an emulsifier,
wherein the cosmetic composition is an emulsion.

2. A cosmetic composition according to claim 1, wherein the cosmetic composition contains the following preservatives in amounts no greater than the following amounts, or wherein the cosmetic composition does not contain any of the following preservatives:
Benzoic acid no greater than 0.062 % wt; Sodium benzoate no greater than 0.062 % wt; Salts of benzoic acid excluding sodium benzoate no greater than 0.003 % w/v; Propionic acid and salts thereof no greater than 0.0032 % w/v; Salicylic acid and salts thereof no greater than 1 % w/v; Hexa-2,4-dienoic acid and salts thereof no greater than 0.4 % w/v; Formaldehyde no greater than 0.01 % w/v; Paraformaldehyde no greater than 0.01 % w/v; Biphenyl-2-ol and salts thereof no greater than 1 x 10⁻⁵ % w/v; Pyrithione zinc no greater than 1.6 x 10⁻⁴ % w/v; Inorganic sulphites no greater than 1.6 x 10⁻⁵ % w/v; Inorganic sulphites no greater than 1.6 x 10⁻⁵ % w/v; Hydrogen sulphites no greater than 1.6 x 10⁻⁵ % w/v; Chlorobutanol no greater than 0.25 % w/v; 4-Hydroxybenzoic acid and salts thereof and esters thereof no greater than 1.2 x 10⁻² % w/v; 3-Acetyl-6-methylpyran-2,4(3H)-dione and salts thereof no greater than 1 x 10⁻³ % w/v; Formic acid no greater than 6 x 10⁻³ % w/v; Sodium formate no greater than 6 x 10⁻³ % w/v; Methenamine 3-chloroallylochloride no greater than 5 x 10⁻³ % w/v; 1-(4-Chlorophenoxy)-1-(imidazole-1-yl)-3,3-dimethylbutan-2-one no greater than 6.3 x 10⁻⁵ % w/v; 1,3-Bis (hydroxymethyl)-5,5-dimethylimidazolidine-2,4-dione no greater than 6.3 x 10⁻⁵ % w/v; Benzyl alcohol no greater than 0.0625 % w/v; 1-Hydroxy-4-methyl-6-(2,4,4-trimethylpentyl) 2-pyrindon no greater than 1.6 x 10⁻⁵ % w/v; Monoethanolamine salt of 1-Hydroxy-4-methyl-6-(2,4,4-trimethylpentyl) 2-pyrindon no greater than 1.6 x 10⁻⁵ % w/v; 2,2'-methylenebis(6-bromo-4-chlorophenol) no greater than 2 x 10⁻³ % w/v; 4-Isopropyl-m-cresol no greater than 0.1 % w/v; 5-Chloro-2-methyl-isothiazol-3(2H)-one no greater than 1.6 x 10⁻³ % w/v; 2-methyl-isothiazol-3(2H)-one no greater than 1.6 x 10⁻³ % w/v; 2-Benzyl-4-chlorophenol no greater than 0.1 % w/v; 2-Chloroacetamide no greater than 6.25 x 10⁻³ % w/v; N,N"-bis(chlorophenyl)-3,12-diamino-2,4,11,13-tetraazatetradecadiamidine no greater than 5 x 10⁻⁴ % w/v; digluconate of N,N"-bis(chlorophenyl)-3,12-diamino-2,4,11,13-tetraazatetradecadiamidine no greater than 5 x 10⁻⁴ % w/v; diacetate of N,N"-bis(chlorophenyl)-3,12-diamino-2,4,11,13-tetraazatetradecadiamidine no greater than 5 x 10⁻⁴ % w/v; dihydrochloride of N,N"-bis(chlorophenyl)-3,12-diamino-2,4,11,13-tetraazatetradecadiamidine no greater than 5 x 10⁻⁴ % w/v; 1-Phenoxypropanol-2-ol no greater than 0.1 % w/v; Alkyl(C₁₂₋₂₂)trimethyl ammonium bromide no greater than 6.1 x 10⁻³ % w/v; Alkyl(C₁₂₋₂₂)trimethyl ammonium chloride no greater than 6.1 x 10⁻³ % w/v; 4,4-Dimethyl-1,3-oxazolidine no greater than 3 x 10⁻⁶ % w/v; N-(hydroxymethyl)-N-(dihydroxymethyl-1,3-dioxo-2,5-imidazolidinyl-4)-N'(hydroxymethyl) urea no greater than 3 x 10⁻⁵ % w/v; Benzenecarboximidamide, 4,4'-(1,6-hexanediylbis (oxy))bis- no greater than 0.116 % w/v; Salts of Benzenecarboximidamide, 4,4'-(1,6-hexanediylbis (oxy))bis- no greater than 0.116 % w/v; Glutaraldehyde (pentane-1,5-dial) no greater than 5 x 10⁻⁴ % w/v; 5-Ethyl-3,7-dioxa-1-azabicyclo[3.3.0] octane no greater than 1.5 x 10⁻⁴ % w/v; 3-(p-Chlorophenoxy)-propane-1,2 diol no greater than 1.25 % w/v; Sodium hydroxymethylamino acetate no greater than 2 x 10⁻⁵ % w/v; Silver chloride no greater than 3.12 x 10⁻³ % w/v; Benzalkonium chloride no greater than 4 x 10⁻³ % w/v; Benzalkonium bromide no greater than 4 x 10⁻³ % w/v; Benzalkonium saccharinate no greater than 4 x 10⁻³ % w/v; Phenylmethoxy methanol no greater than 3 x 10⁻⁵ % w/v; and 3-lodo-2-propynylbutylcarbamate no greater than 6.25 x 10⁻³ % w/v.

3. A cosmetic composition according to claim 1 or 2, wherein water is present in an amount of from 5 to 20% by weight of the total composition;

4. A cosmetic composition according to any one of the preceding claims, wherein saturated fats are present in an amount of 2% to 10% by weight of the total composition.

5. A cosmetic composition according to any one of the preceding claims, wherein the saturated fats are provided by mango butter, coconut oil, Japan wax, avocado butter and mixtures thereof.

6. A cosmetic composition according to any one of the preceding claims, wherein salt is present in an amount of 30% to 70% by weight of the total composition.

7. A cosmetic composition according to any one of the preceding claims, wherein salt is present in an amount of 45% to 55% by weight of the total composition.

8. A cosmetic composition according to any one of the preceding claims, wherein the emulsifier is present in an amount of from 1 to 20% by weight of the total composition.

9. A cosmetic composition according to any one of the preceding claims, wherein the emulsifier is present in an amount of from 5 to 15% by weight of the total composition.

10. A cosmetic composition according to any one of the preceding claims, wherein the emulsifier is selected from triethanolamine, lactic acid, stearic acid, cetearyl alcohol, glyceryl stearate, cetyl alcohol, sodium laureth sulfate, glyceryl stearate, polyethylene glycol and mixtures thereof.

11. A cosmetic composition according to any one of the preceding claims, wherein the emulsifier is selected from stearic acid, cetearyl alcohol and mixtures thereof.

12. A cosmetic composition according to any one of the preceding claims further comprising glycerine.

13. A cosmetic composition according to claim 12, wherein the glycerine is present in an amount of from 5% to 20% by weight of the total composition.

14. A cosmetic composition according to any one of the preceding claims further comprising a surfactant in an amount of from 1 to 20% by weight of the total composition.

15. A cosmetic composition according to claim 14, wherein the surfactant is selected from sodium lauryl sulphate, sodium laureth sulphate, ammonium lauryl sulphate, ammonium laureth sulphate, sodium cocoamphoacetate, disodium laureth sulfosuccinate, lauryl betaine and mixtures thereof.
